# FASCICULE DE BREVET EUROPEEN

(11) **EP 1 355 887 B1**
(45) Date de publication et mention de la délivrance du brevet: **15.08.2007**
(21) Numéro de dépôt: 02703583.1
(22) Date de dépôt: 25.01.2002
(51) Int. Cl.: C07D 239/54, A61K 31/505

(54) **DERIVES D'ACYCLONUCLEOSIDES PYRIMIDINIQUES, LEUR PROCEDE DE PREPARATION ET LEUR UTILISATION**
PYRIMIDINISCHE AZYKLONUKLEOSIDE, VERFAHREN ZUR IHRER HERSTELLUNG UND IHRE VERWENDUNG
PYRIMIDINE ACYCLONUCLEOSIDE DERIVATIVES, PREPARATION METHOD AND USE THEREOF

(30) Priorité: 29.01.2001 FR 0101165; 19.06.2001 FR 0108052
(43) Date de publication de la demande: 29.10.2003
(73) Titulaire: UNIVERSITE DE GENEVE, 1211 Geneve 4 (CH)
(72) Inventeur: TRONCHET, Jean, M., J., F-01220 Grilly (FR)
(74) Mandataire: Pautex Schneider, Nicole Véronique
(86) Numéro de dépôt international: PCT/EP2002/000839
(87) Numéro de publication internationale: WO 2002/060880

(56) Documents cités:
- EP-A- 0 420 763
- EP-A- 0 631 783
- DATABASE CHEMABS [en ligne] CHEMICAL ABSTRACTS SERVICE, COLUMBUS, OHIO, US; TRONCHET, JEAN M. J. ET AL: "Antiviral nucleoside N-hydroxyureas and carbamates" retrieved from STN Database accession no. 127:346601 XP002172470 & CARBOHYDR. LETT. (1997), 2(5), 313-320, cité dans la demande
- PONTIKIS, RENEE ET AL: "Synthesis and Anti-HIV Activity of Novel N-1 Side Chain-Modified Analogs of 1-[(2-Hydroxyethoxy)methyl]-6-(phenylthio) thymine (HEPT)" J. MED. CHEM. ( 1997 ), 40(12), 1845 -1854, XP002172467
- TRONCHET JMJ GRIGOROV M DOLATSHAHI N MORIAUD F WEBER J: "A QSAR study confirming the heterogeneity of the HEPT derivative series regarding their interaction with HIV reverse transcriptase" EUROPEAN JOURNAL OF MEDICINAL CHEMISTRY.CHIMICA THERAPEUTICA,FR,EDITIONS SCIENTIFIQUE ELSEVIER, PARIS, vol. 32, no. 4, 1997, pages 279-299, XP004086652 ISSN: 0223-5234 cité dans la demande
- LUCO, JUAN M. ET AL: "QSAR Based on Multiple Linear Regression and PLS Methods for the Anti-HIV Activity of a Large Group of HEPT Derivatives" J. CHEM. INF. COMPUT. SCI. ( 1997 ), 37(2), 392 -401, XP002172468
- JALALI-HERAVI, M. ET AL: "Use of Artificial Neural Networks in a QSAR Study of Anti-HIV Activity for a Large Group of HEPT Derivatives" J. CHEM. INF. COMPUT. SCI. ( 2000 ), 40(1), 147 -154, XP002172469

## Description

### Domaine technique

La présente invention concerne des dérivés d'acyclonucléosides pyrimidiniques actifs comme inhibiteurs non nucléosidiques de la transcriptase inverse du VIH-1, leur procédé de préparation et leur utilisation.

### Etat de la technique

La tendance actuelle dans le traitement du SIDA consiste à faire appel à des polythérapies n'incluant plus les antiprotéases dont les effets secondaires sont gênants et les modalités de prise du médicament contraignantes pour le patient.

Les polythérapies préconisées comportent donc un ou deux inhibiteurs nucléosidiques auxquels sont associés un ou deux inhibiteurs non nucléosidiques [G. J. Moyle, Infect. Med. 17(6) 442-455 (2000)].

Il est donc important de développer de nouveaux inhibiteurs allostériques de la transcriptase inverse pourvus d'une grande activité spécifique et d'une faible toxicité.

Des inhibiteurs allostériques de la transcriptase inverse à structure d'acyclonucléosides sont décrits par exemples dans les demandes de brevet EP-A-0 449 726 , WO-A-97/43266, WO-A-97/30979, WO-A-96/16675, WO-A-95/18109, EP-A-0 631 783, EP-A-0 420 763, l'exemple le plus connu étant l' EMIVIRINE ayant la Formule A suivante :

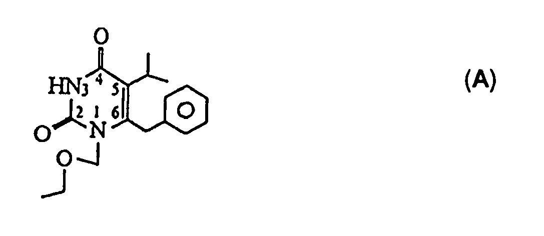

Ces inhibiteurs allostériques de la transcriptase inverse à structure d'acyclonucléosides agissent à des concentrations nanomolaires et ils ont le désavantage de sélectionner très rapidement des mutants résistants.

D'autre part, il a été montré par J. M. J. Tronchet, M. Zsély, M. Iznaden, F. Barbalat-Rey, M. Geoffroy & G. Bernardinelli, Carbohydr. Lett. 2, 101-108 (1996) en relation avec le composé de Formule **B** suivante : 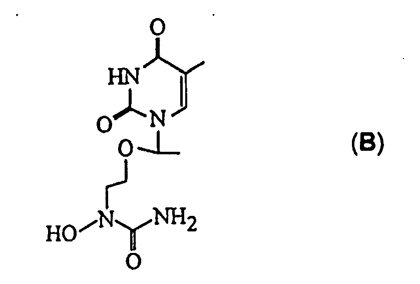 qu'un reste *N*-hydroxyuréido fixé en ω sur le radical porté par N-1 d'une thymine se comportait comme une « pseudo-nucléobase » contractant dans le cristal deux liaisons hydrogène (par son groupe CONH₂) avec la nucléobase d'une autre molécule tandis que le groupe N-OH fonctionnait comme donneur de liaison hydrogène vis-à-vis de l'atome d'oxygène d'un alcool.

Le groupement *N*-hydroxyuréido pouvait donc constituer un candidat intéressant pour contracter de nouvelles liaisons avec le site allostérique de la transcriptase inverse et éventuellement à l'extérieur de celui-ci avec des nucléobases des acides nucléiques « traités » par l'enzyme.

En outre, du point de vue de la cytotoxicité des produits, le groupement *N*-hydroxyuréido pouvait également se révéler un bon candidat car une étude structure-activité d'acyclonucléosides actifs contre le VIH-1 avait montré qu'un groupement hydrophile fixé en N-1 sur la nucléobase devait diminuer la cytotoxicité du composé [J. M. J. Tronchet, M. Grigorov, N. Dolatshahi, F. Moriaud & J. Weber, Eur. J. Med. Chem., 32, 279-299 (1997)].

Ainsi, en introduisant un groupe *N*-hydroxyuréido en position ω de la chaîne fixée en N-1 d'une pyrimidine substituée en C-4 et C-5, on pouvait espérer améliorer l'activité anti-VIH-1 des produits analogues déjà connus.

Cependant, le groupement *N*-hydroxyuréido ne s'est pas révélé suffisant pour assurer une activité puisque le premier composé préparé, à savoir le composé ayant la Formule **B** ci-dessus s'est révélé totalement inactif contre VIH-1 et le second composé préparé, à savoir le composé ayant la Formule **C** suivante [J. M. J. Tronchet, M. lznaden, & N. Laroze, Carbohydr. Lett. 2, 313-320 (1997)] s'est révélé très modérément actif (Cl₅₀ 70 nM).

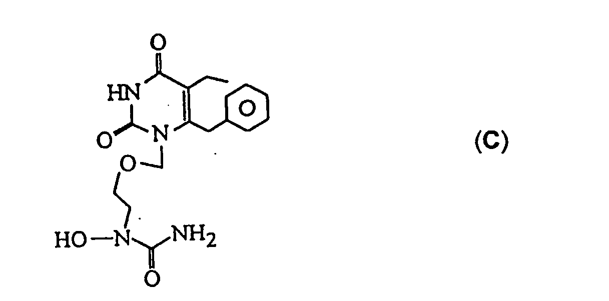

Sur la base de ces faits, les présents inventeurs ont poursuivi leurs recherches et ils ont trouvé avec surprise qu'en rallongeant la chaîne située entre la nucléobase et le groupe *N*-hydroxyuréido de un atome de carbone pour passer d'une chaîne à deux atomes de carbone à une chaîne à trois atomes de carbone, on pouvait obtenir un acyclonucléoside atoxique et actif contre VIH-1 à des concentrations bien plus faibles que celles atteintes jusqu'alors avec des produits analogues déjà connus, alors que la même homologation effectuée sur l'analogue du composé de Formule **C** portant un groupe hydroxyle à la place du groupe N-hydroxyuréido [cf. N. Laroze, Université de Genève, Thèse No. 3212 (2000)] diminuait sensiblement l'activité.

La présente invention a été réalisée sur la base de ces résultats.

### Exposé de l'invention

La présente invention a pour objet un composé de Formule générale **I** suivante : 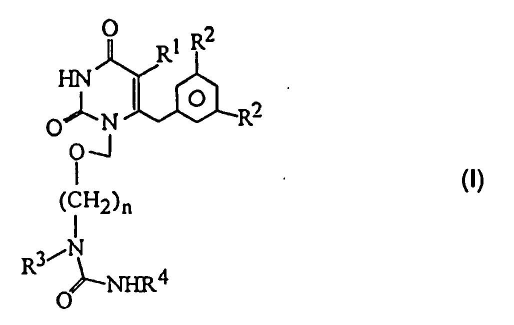 dans laquelle :
- n est égal à 3;
- R¹ représente un groupe éthyle ou un groupe isopropyle;
- chacun des groupes R² représente indépendamment l'un de l'autre un atome d'hydrogène, un groupe alkyle en C₁-C₃, ou un atome d'halogène;
- l'un des groupes R³ et R⁴ représente un atome d'hydrogène alors que l'autre des groupes R³ et R⁴ représente un groupe -OH ou -OR⁵, où R⁵ peut être un groupe acyle en C₂-C₇, un groupe alkyl(en C₁-C₆)aminocarbonyle, un groupe aralkyl(C₁-C₆)aminocarbonyle éventuellement substitué sur l'aryle, un groupe arylcarbonyle éventuellement substitué ou un groupe hétéroarylaminocarbonyle,
ou un sel pharmaceutiquement acceptable de celui-ci.

La présente invention a également pour objet :
- un composé de Formule **I** tel que défini ci-dessus pour utilisation comme médicament;
- un composé de Formule **I** tel que défini ci-dessus pour utilisation comme agent antiviral, notamment comme agent anti-VIH-1;
- un procédé de préparation d'un composé de Formule I tel que défini ci-dessus;
- une composition pharmaceutique contenant en tant qu'ingrédient actif au moins un composé de Formule **I** tel que défini ci-dessus;
- une composition pharmaceutique contenant une quantité efficace comme antiviral d'un composé de Formule **I** tel que défini ci-dessus; et
- l'utilisation d'un composé de Formule **I** tel que défini ci-dessus pour la fabrication d'un médicament anti-VIH-1;
étant entendu que par "composé de Formule I tel que défini ci-dessus", il faut également comprendre un sel pharmaceutiquement acceptable de celui-ci.

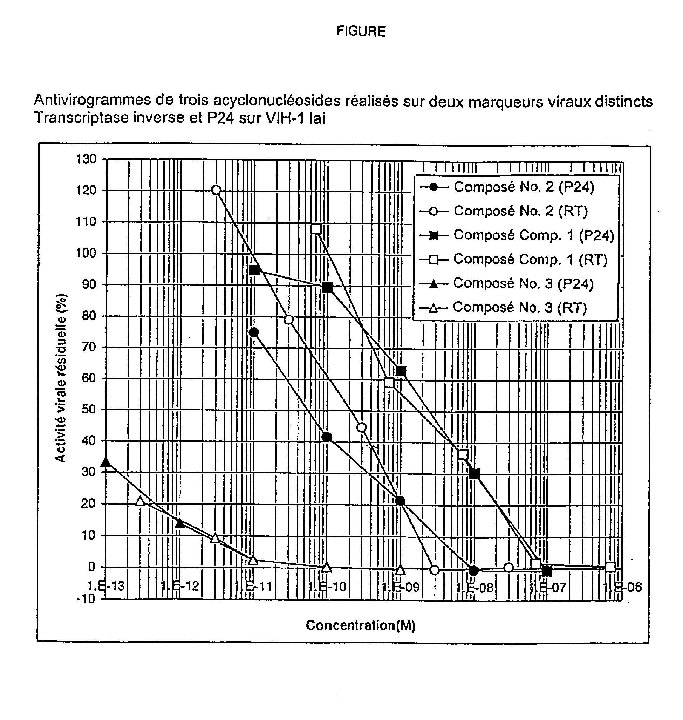

### Description de la Figure

La Figure représente des antivirogrammes de trois acyclonucléosides réalisés sur deux marqueurs antiviraux, à savoir la transcriptase inverse et P24, pour évaluer l'activité antirétrovirale de ces trois acyclonucléosides sur VIH-1 lai.

### Description détaillée de l'invention.

Le composé de la présente invention est représenté par la Formule générale **I** suivante : 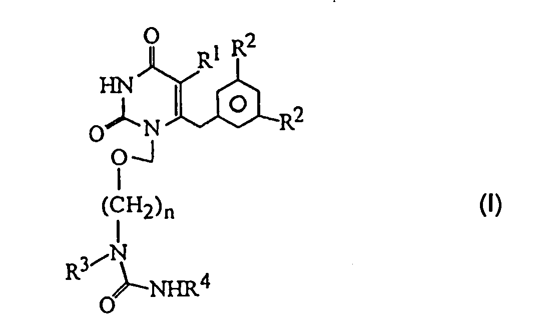 dans laquelle n est égal à 3.

Le groupe R¹ peut être soit un groupe éthyle soit un groupe isopropyle mais de préférence, le groupe R¹ est un groupe isopropyle.

Les groupes R² peuvent être chacun indépendamment l'un de l'autre un atome d'hydrogène, un groupe alkyle en C₁-C₃, par exemple un méthyle, un éthyle, un n-propyle ou un isopropyle, ou un atome d'halogène, par exemple un chlore, un brome, un iode ou un fluor.

De préférence, les groupes R² sont identiques et ils représentent chacun un groupe méthyle.

L'un des groupes R³ et R⁴ représente un atome d'hydrogène alors que l'autre des groupes R³ et R⁴ représente un groupe -OH ou un groupe -OR⁵, où R⁵ est un groupe protecteur du groupe hydroxy.

Ainsi, lorsque le groupe R³ est un groupe -OH ou un groupe -OR⁵ , le groupe R⁴ est un atome d'hydrogène; et lorsque le groupe R³ est un atome d'hydrogène, le groupe R⁴ est un groupe -OH ou un groupe -OR⁵.

Cependant, il est préférable que le groupe R³ soit un groupe -OH ou un groupe -OR⁵ et que le groupe R⁴ soit un atome d'hydrogène.

Le groupe R⁵ doit être choisi parmi les groupes de protection de l'hydroxy qui puissent être facilement libérés dans un milieu biologique et qui sont non toxiques.

Le groupe R⁵ est ainsi choisi parmi un groupe acyle en C₂-C₇, un groupe alkyl(en C₁-C₆)aminocarbonyle, un groupe aralkyl(en C₁-C₆)aminocarbonyle éventuellement substitué sur l'aryle, un groupe arylcarbonyle éventuellement substitué ou un groupe hétéroarylaminocarbonyle, étant entendu que le groupe acyle et le groupe alkyle des groupes alkyl(en C₁-C₆ )aminocarbonyle et aralkyl(en C₁-C₆)aminocarbonyle peuvent être ramifiés ou non.

Des exemples du groupe acyle en C₂-C₇ incluent un groupe acétyle, un groupe propionyle, un groupe butyryle ou un groupe pivaloyle, sans être limités à ceux-ci.

Des exemples du groupe alkyl(en C₁-C₆ )aminocarbonyle incluent un groupe méthylaminocarbonyle, un groupe éthylaminocarbonyle, un groupe propylaminocarbonyle, un groupe isopropylaminocarbonyle, un groupe butylaminocarbonyle, un groupe pentylaminocarbonyle ou un groupe hexylaminocarbonyle, sans être limités à ceux-ci.

Des exemples du groupe aralkyl(en C₁-C₆ )aminocarbonyle éventuellement substitué sur l'aryle incluent un groupe benzylaminocarbonyle, un groupe phénéthylaminocarbonyle, un groupe phényl-3-propylaminocarbonyle, un groupe phényl-4-butylaminocarbonyle, un groupe phényl-5-pentylaminocarbonyle ou un groupe phényl-6-hexylaminocarbonyle, ainsi que les groupes aralkyl(en C₁-C₆ )-aminocarbonyle ci-dessus dans lesquels l'aryle porte 1 ou 2 substituants choisis parmi les halogènes, par exemple un chlore, un brome, un fluor ou un iode, les alkyles en C₁-C₃, par exemple un méthyle, un éthyle, un propyle ou un isopropyle et les groupes alcoxy en C₁-C₃, par exemple un méthoxy, un éthoxy, un propoxy ou un isopropoxy, sans être limités à ceux-ci.

Des exemples du groupe arylcarbonyle éventuellement substitué incluent un groupe benzoyle, un groupe p-chlorobenzoyle, un groupe p-méthoxybenzoyle ou un groupe p-nitrobenzoyle, sans être limités à ceux-ci.

Des exemples du groupe hétéroarylaminocarbonyle incluent un groupe isoxazol-3-ylaminocarbonyle, un groupe isoxazol-4-ylaminocarbonyle, un groupe isoxazol-5-ylaminocarbonyle, un groupe pyridin-2-ylaminocarbonyle ou un groupe pyridine-3-ylaminocarbonyle, sans être limités à ceux-ci.

Ces composés de Formule **I** de la présente invention peuvent être sous la forme de sels pharmaceutiquement acceptables conventionnels de ceux-ci.

Les composés de la présente invention peuvent être préparés selon les schémas réactionnels suivants dans lesquels n, R¹, R², R³, R⁴ et R⁵ ont les mêmes significations que celles définies ci-dessus.

Le Schéma réactionnel 1 suivant montre les étapes du procédé de préparation du composé de Formule **I** de la présente invention dans lequel R³ est un groupe -OH ou un groupe -OR⁵ et R⁴ est un atome d'hydrogène :

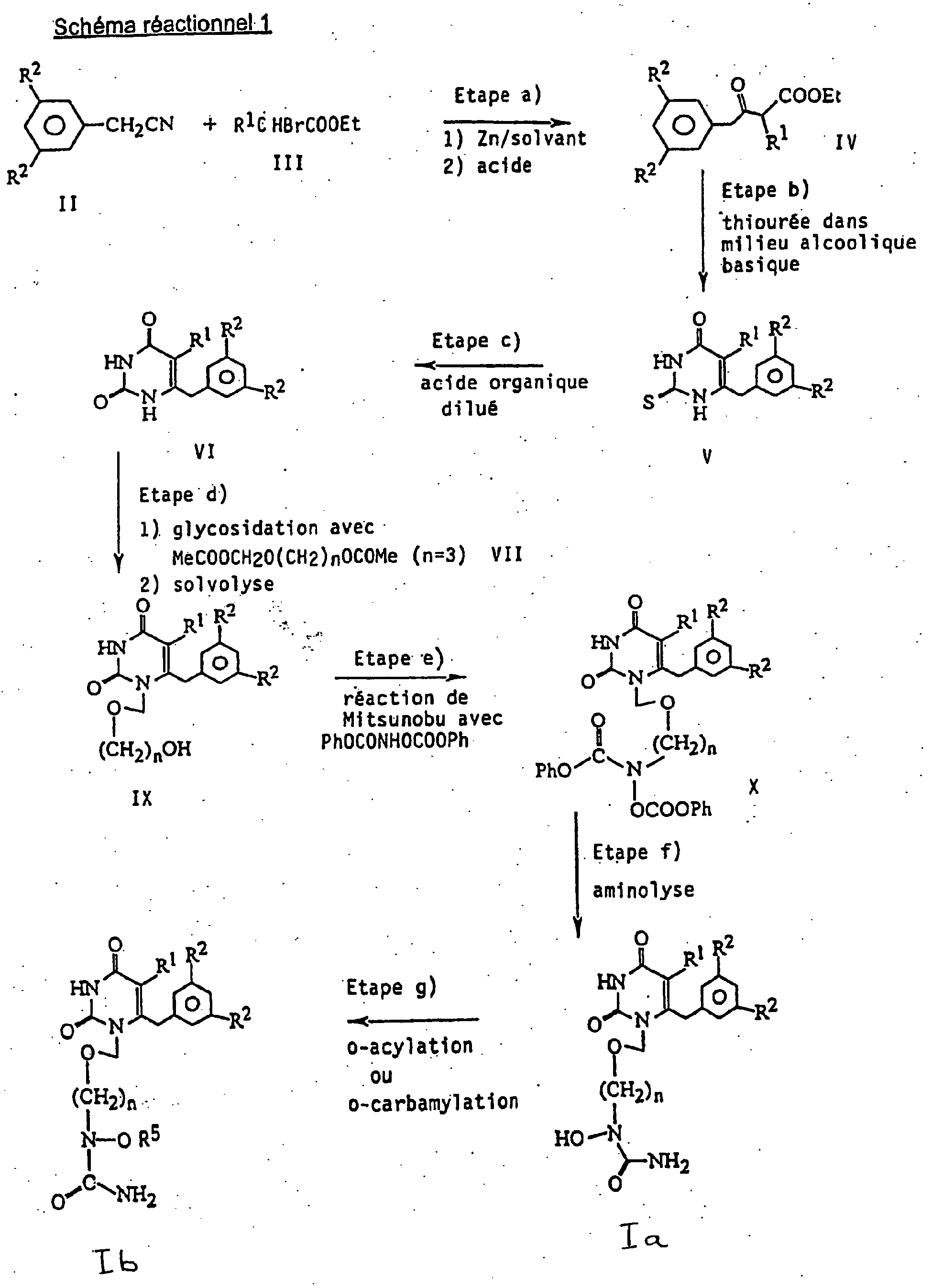

Les nucléobases représentées par les Formules **V** et **VI** ci-dessus sont connues et elles sont habituellement préparées par substitution électrophile [cf., p. ex. Y. S. Lee & Y. H. Kim, Synth. Commun. 29(9) 1503-17 (1999)].

Toutefois, dans le procédé de la présente invention, le cycle pyrimidine des nucléobases modifiées de Formules **V** et **VI** a été construit par condensation selon le principe de la technique décrite dans S.M. Hannick & Y. Kishi, J. Org. Chem., 48, 3833-3835 (1983), ce qui permet d'accéder à une grande variété de bases différant par la nature des groupes R¹ et R²

Ainsi, la première étape du procédé, dite étape a), consiste à faire réagir un phényléthanenitrile de Formule **II** avec un composé de Formule **III,** en présence de Zn dans un solvant approprié, par exemple un éther tel que le tétrahydrofurane, à une température appropriée, par exemple à reflux, puis à traiter le mélange obtenu avec un acide, par exemple l'acide chlorhydrique, pour obtenir le composé de Formule **IV.**

Ensuite, dans une étape b), ce composé de Formule **IV** est mis à réagir avec de la thiourée dans un milieu alcoolique basique, par exemple dans un milieu sodium/éthanol (Na/EtOH), à une température appropriée, par exemple à reflux, pour obtenir le thiouracile de Formule **V**.

Le thiouracile de Formule **V** est ensuite transformé dans une étape c) en uracile de Formule **VI** par traitement avec un acide organique dilué approprié, par exemple de l'acide chloroacétique 10 %.

La fixation de la chaîne sur N¹ de la nucléobase de Formule **VI** est effectuée dans une étape d) par une réaction de glycosidation conventionnelle avec un composé de Formule **VII** dans lequel n est égal à 3, par exemple en présence d'hexaméthyldisilazane (HMDS), de chlorotriméthylsilane (TMSCI) et de tétrachlorure d'étain (SnCl₄) suivie d'une solvolyse de l'ester obtenu, par exemple dans un milieu méthanol/triéthylamine (MeOH/Et₃N), pour fournir l'alcool de Formule **IX.**

L'alcool de Formule **IX** est ensuite soumis à une réaction de Mitsunobu pour remplacer son groupement -OH par un groupement azoté qui conduira au groupe *N*-hydroxyuréido.

Ainsi, le traitement de l'alcool de Formule **IX** dans les conditions d'une réaction de Mitsunobu conventionnelle en utilisant la *N,*O-*bis*(phénoxycarbonyl)hydroxylamine comme nucléophile (étape e) fournit le composé de Formule **X** qui, après aminolyse conventionnelle (étape f), par exemple avec de l'ammoniac ou un amidure dans un solvant approprié, conduit au composé de Formule **Ia** de la présente invention qui porte un groupe *N*¹-hydroxyuréido terminal, à savoir un composé de la présente invention de Formule **I** dans lequel R³ représente un groupe -OH et R⁴ représente un atome d'hydrogène.

Le composé de Formule **Ia** peut ensuite être transformé par une réaction d'O-acylation ou d'O-carbamylation (étape g) en un composé de la présente invention de Formule **Ib,** à savoir un composé de la présente invention de Formule **I** dans lequel R³ représente un groupe -OR⁵ et R⁴ représente un atome d'hydrogène, comme suit.

Le composé de Formule **Ia** de la présente invention peut être soumis à une réaction d'O-acylation conventionnelle, par exemple avec un agent acylant conventionnel de formule générale X-CO-R⁶ (où R⁶ est un groupe alkyle en C₁-C₆ qui peut être ramifié ou non, ou un groupe aryle éventuellement substitué, X pouvant être par exemple un atome d'halogène ou un groupe R⁶COO-, sans que X soit limité à ceux-ci), pour conduire au composé monoacylé de Formule **Ib** de la présente invention dans lequel R⁵ est un groupe acyle en C₂-C₇ ou un groupe arylcarbonyle éventuellement substitué, plus lipophile que son précurseur la et dans lequel le groupe N-OH est protégé contre l'oxydation.

Le composé de Formule **Ia** de la présente invention peut également être soumis à une réaction d'O-carbamylation conventionnelle, par exemple avec un agent de carbamylation conventionnel de formule générale X-CO-NH-R⁷ (où R⁷ est un groupe alkyle en C₁-C₆ qui peut être ramifié ou non, un groupe aralkyle en C₁-C₆ éventuellement substitué sur l'aryle et dont le groupe alkyle peut être ramifié ou non, ou un groupe hétéroaryle, X pouvant être par exemple un atome d'halogène, sans que X soit limité à ceux-ci) pour conduire au composé de la présente invention de Formule **Ib** dans lequel R⁵ est un groupe alkyl(en C₁-C₆) aminocarbonyle, un groupe aralkyl(en C₁-C₆)aminocarbonyle éventuellement substitué sur l'aryle, ou un groupe hétéroarylaminocarbonyle, plus lipophile que son précurseur **Ia** et dans lequel le groupe N-OH est protégé contre l'oxydation.

Pour obtenir un composé de la présente invention de Formule **I** dans lequel R³ est un atome d'hydrogène et R⁴ est un groupe -OH ou un groupé -OR⁵, on se réfèrera au Schéma réactionnel 2 suivant :

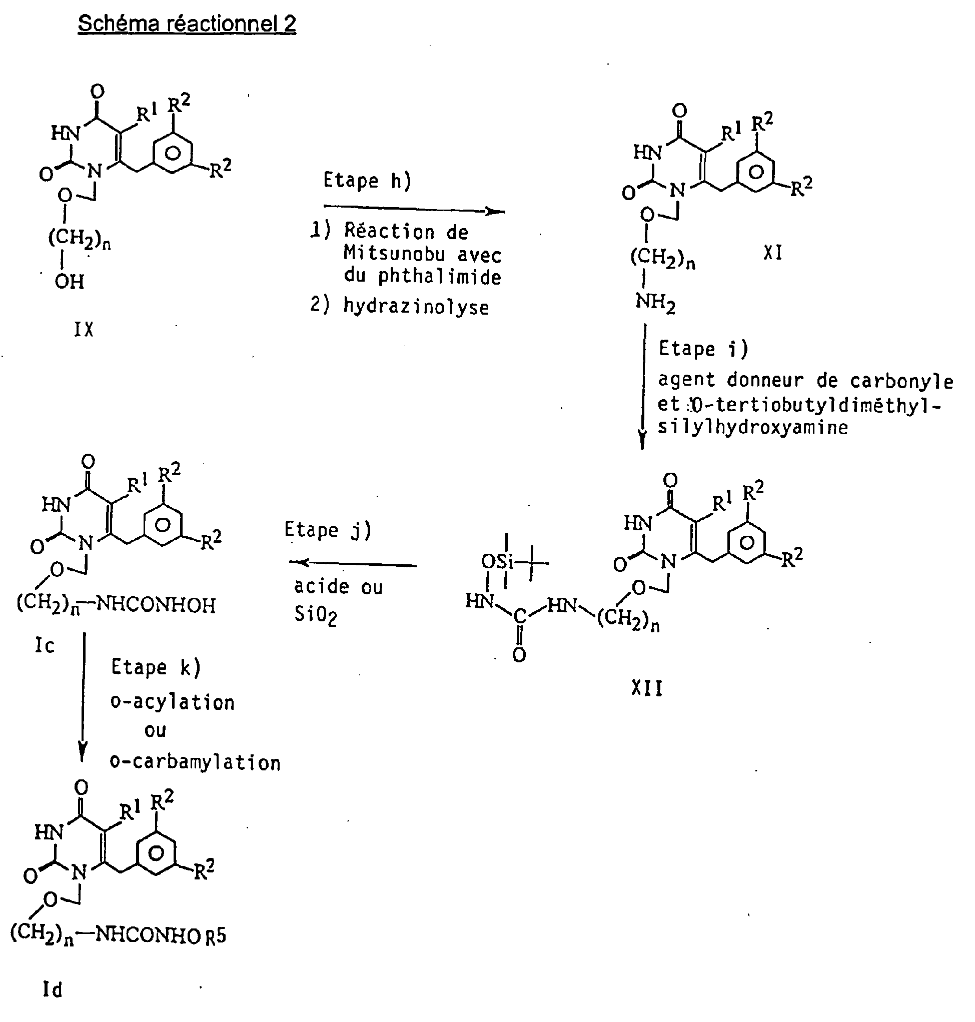

L'alcool de Formule **IX** obtenu comme dans les étapes a) à d) décrites ci-dessus en référence au Schéma réactionnel 1 est soumis, dans une étape h), à une réaction de Mitsunobu conventionnelle en utilisant le phtalimide comme nucléophile, suivie d'une hydrazinolyse conventionnelle pour conduire à l'amine de Formule **XI.**

Ensuite, dans une étape i), cette amine de Formule **XI** est traitée de manière conventionnelle par un agent donneur de carbonyle, par exemple le carbonyldiimidazole ou le phosgène, et la *O*-tertiobutyldiméthylsilylhydroxylamine fournit le composé de Formule **XII.**

Ce composé de Formule **XII** est ensuite soumis à une réaction de dé-*O*-silylation (étape j), par exemple dans un milieu acide ou en présence de SiO₂, pour fournir le composé de la présente invention de Formule **Ic** porteur d'un groupe *N*³-hydroxyuréido terminal, à savoir le composé de la présente invention de Formule **I** dans lequel R³ est un atome d'hydrogène et R⁴ est un groupe -OH.

Le composé de Formule **Ic** de la présente invention peut ensuite être transformé par une réaction d'O-acylation ou d'O-carbamylation (étape k) en un composé de la présente invention de Formule **Id,** à savoir un composé de la présente invention de Formule **I** dans lequel R³ représente un atome d'hydrogène et R⁴ représente un groupe -OR⁵, comme suit.

Le composé de Formule **Ic** de la présente invention peut être soumis à une réaction d'O-acylation conventionnelle, par exemple avec un agent acylant conventionnel de formule générale X-CO-R⁶ (où R⁶ est un groupe alkyle en C₁-C₆ ramifié ou non, ou un groupe aryle éventuellement substitué, X pouvant être par exemple un atome d'halogène ou un groupe R⁶COO-, sans que X soit limité à ceux-ci) pour conduire au composé monoacylé de Formule **Id** de la présente invention dans lequel R⁵ est un groupe acyle en C₂-C₇ ou un groupe,arylcarbonyle éventuellement substitué.

Le composé de Formule **Ic** de la présente invention peut également être soumis à une réaction d'O-carbamylation conventionnelle, par exemple avec un agent de carbamylation conventionnel de formule générale X-CO-NH-R⁷ (où R⁷ est un groupe alkyle en C₁-C₆, ramifié ou non, un groupe aralkyle en C₁-C₆ éventuellement substitué sur l'aryle et dont le groupe alkyle peut être ramifié ou non, ou un groupe hétéroaryle, X pouvant être par exemple un halogène, sans que X soit limité à ceux-ci) pour conduire au composé de la présente invention de Formule **Id** dans lequel R⁵ est un groupe alkyl(en C₁-C₆) aminocarbonyle, un groupe aralkyl(en C₁-C₆)aminocarbonyle éventuellement substitué sur l'aryle, ou un groupe hétéroarylaminocarbonyle, plus lipophile que son précurseur **Ic** et dans lequel le groupe N-OH est protégé contre l'oxydation.

Les composés de la présente invention peuvent en outre être transformés de manière conventionnelle en sels pharmaceutiquement acceptables de ceux-ci.

Afin de démontrer l'efficacité des composés de la présente invention comme agents antiviraux, et notamment comme agent anti-VIH-1, des tests *in vitro* ont été effectués selon le principe et le mode opératoire suivants.

### Principe

Le criblage a pour but d'évaluer l'activité antirétrovirale des composés de la présente invention et des composés comparatifs sur la souche VIH-1 lai cultivée sur cellules mononuclées du sang périphérique (CMSP).

Pour cela, les composés à tester ont été incubés pendant sept jours (durée du test anti-VIH) avec les CMSP en phase exponentielle de croissance.

La capacité des composés à inhiber la réplication virale a ensuite été mesurée dans les surnageants de culture soit par dosage de l'activité "Reverse Transcriptase" (RT) à l'aide de la trousse RetroSys TR (INNOVAGEN) soit par dosage de la protéine P24 à l'aide de la trousse Innotest HIV Antigen mAb Screening (IMMUNOGENETICS).

Les concentrations du composé testé permettant d'inhiber de 50 et 90 % la réplication virale ont été déterminées. Il s'agit respectivement des concentrations d'inhibition Cl₅₀ et Cl₉₀.

Parallèlement au criblage, et dans les mêmes conditions, un test de cytotoxicité des composés à tester a été effectué.

Ce test a été révélé par le MTT (bromure de 3-[4,5-diméthylthiazol-2-yl]-2,5-diphenyltetrazolium) après 7 jours de culture [J.G. Park et col., Cancer Res. 47(22), 5875-5879 (1987)].

Ce test a permis de déterminer la concentration de produit qui diminue de 50 % la croissance et la viabilité des CMSP (CC₅₀).

L'indice thérapeutique (IT) du composé a été calculé comme suit : IT₅₀ = (CC₅₀/Cl₅₀).

### Mode opératoire

Les CMSP ont été isolées comme décrit dans Ulmer et col., Immunobiology, 166(3), 238-250 (1984) à partir d'un donneur sain par un gradient de Ficoll (Nicomed Pharma AS).

Les CMSP ont été activées par la phytohémagglutinine pendant trois jours puis cultivées dans une plaque de 96 puits, dans du milieu de base (RPMI) supplémenté par de l'interleukine-2 humaine recombinante.

Tout au long de la culture, les cellules ont été maintenues à 37°C, en atmosphère saturée en humidité, et sous 5 % de CO₂.

Les CMSP ont été pré-traitées une heure en présence des composés à tester à différentes concentrations, puis infectées avec 100 TCID₅₀ de VIH-1-Lai [S. Wain-Hobson et col., Science 17, 252 (5008) : 961-965 (1991)].

Trois jours après l'infection par le VIH-1, la moitié du milieu de culture a été renouvelée et au jour 7, les surnageants ont été prélevés et congelés à -20°C.

La réplication virale a ensuite été mesurée :
a) en dosant l'activité "Reverse Transcriptase" (RT) dans les surnageants de culture au moyen de la trousse RetroSys (Innovagen).
   La RT contenue dans le surnageant synthétise, en présence de bromodésoxyuridine triphosphate (BrdUTP), un brin d'ADN complémentaire à une matrice fixée au fond des puits d'une plaque 96 puits. L'incorporation de BrdU est quantifiée par la fixation d'un anticorps anti-BrdU conjugué à la phosphatase alcaline. L'activité de la phosphatase alcaline liée, mesurée par colorimétrie, est proportionnelle à l'activité de la RT dans le surnageant de culture.
b) en dosant la protéine P24 dans les surnageants de culture au moyen de la trousse Innotest HIV Antigen mAb Screening (IMMUNOGENETICS).
   La P24 des surnageants se lie à des anticorps polyclonaux anti-VIH fixés au fond des puits d'une plaque 96 puits. Un anticorps secondaire biotinylé anti-P24 se fixe à la P24. Ensuite, la biotine fixe la streptavidine dont l'activité, mesurée par colorimétrie, est proportionnelle à la quantité de P24 présente dans le surnageant de culture.

Les résultats présentés résultent d'au moins deux expérimentations distinctes et réalisées en triplicat.

### Composés de l'invention testés :

Composé 1: 1-(3-N³-hydroxyuréidopropyloxyméthyl)-6-(3,5-diméthylbenzyl)-5-éthyluracile
Composé 2 : 1-(3-N¹-hydroxyuréidopropyloxyméthyl)-6-(3,5-diméthylbenzyl)-5-éthyluracile
Composé 3 : 1-(3-N¹-hydroxyuréidopropyloxyméthyl)-6-(3,5-diméthylbenzyl)-5-isopropyluracile
Composé 4 : 1-(3-*N*¹-acétoxyuréidopropyloxyméthyl)-6-(3,5-diméthylbenzyl)-5-isopropyluracile.
Composé 5 : 1-(3-*N*¹-pivaloyloxyuréidopropyloxyméthyl)-6-(3,5-diméthylbenzyl)-5-isopropyluracile.

### Composés comparatifs testés :

Composé Comp. 1 : 1-(2-N¹-hydroxyuréidoéthyloxyméthyl)-6-(3,5-diméthylbenzyl)-5-éthyluracile
Composé Comp. 2 : 1-(4-N¹-hydroxyuréidobutyloxyméthyl)-6-(3,5-diméthylbenzyl)-5-éthyluracile
Composé Comp. 3 : 1-(2-N¹-hydroxyuréidoéthyloxyméthyl)-6-benzyl-5-éthyluracile (Composé de Formule C)
Composé Comp. 4 : 1-éthyloxyméthyl-6-benzyl-5-isopropyluracile. (Composé de Formule **A**)

Les composés Nos. 1, 2, 3, 4 et 5 de la présente invention et les composés comparatifs Comp. 1, Comp. 2 et Comp. 3 testés ont été synthétisés comme indiqué dans les Exemples et le composé comparatif Comp. 4 a été synthétisé comme décrit dans la littérature [M. Baba, H. Tanaka, T. Miyasaka, S. Yuseda, M. Ubasawa, R.T. Walker & E. De Clerq, Nucleosides Nucleotides, 14, 575-583 (1995)].

### Résultats des tests

Les résultats des tests résultant des dosages de l'activité "Reverse transcriptase" et de la protéine P24 avec les Composés Nos. 2 et 3 de la présente invention et le composé comparatif Comp. 1 sont présentés sur les antivirogrammes représentés sur la Figure.

Ces résultats montrent clairement que les composés testés inhibent de manière parallèle la production de deux marqueurs viraux distincts, à savoir la transcriptase inverse et la protéine P24.

Ces composés sont donc des inhibiteurs de la replication du virus VIH-1.

Les résultats des tests résultant du dosage de l'activité "Reverse Transcriptase" (RT) sont reportés sur le Tableau 1 ci-dessous qui montre les concentrations d'inhibition à 50% (Cl₅₀) et à 90% (Cl₉₀) contre le VIH-Lai en nM, les concentrations cytotoxiques à 50% (CC₅₀) en nM et l'index thérapeutique IT₅₀ (CC₅₀/Cl₅₀) des Composés Nos. 1, 2, 3, 4 et 5 de la présente invention et des composés comparatifs Comp. 1, Comp. 2, Comp. 3 et Comp. 4.

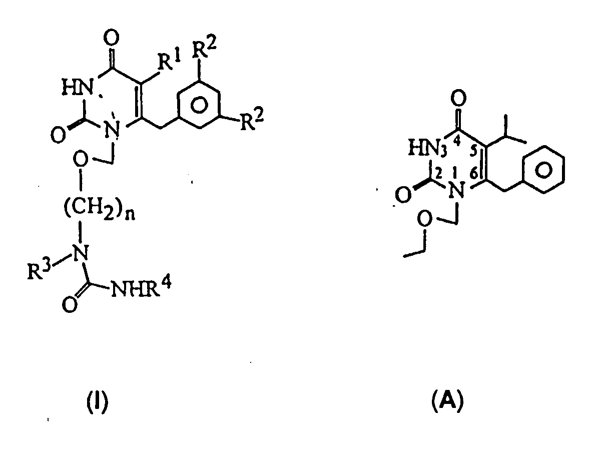

**Tableau 1**

| | R¹ | R² | n | R³ | R⁴ | Cl₅₀ (nM) | Cl₉₀ (nM) | CC₅₀ (nM) | IT₅₀ |
|---|---|---|---|---|---|---|---|---|---|
| Composés de l'invention | | | | | | | | | |
| 1 | Et | Me | 3 | H | OH | 2.3 | 9 | 2,6 x 10⁵ | 1,13 X10⁵ |
| 2 | Et | Me | 3 | OH | H | 0.24 | 1.7 | >3 x 10⁵ | >10⁶ |
| 3 | iPr | Me | 3 | OH | H | 3x10⁻⁵ | 3,9 10⁻³ | 2,2 x 10⁵ | 7x10⁹ |
| 4 | iPr | Me | 3 | OAc | H | 0,03 | 0,6 | 3x10⁴ | 10⁶ |
| 5 | iPr | Me | 3 | OOCCMe₃ | H | 1x10⁻⁶ | | 6,5x10⁴ | 6,5x10¹⁰ |

| Composés Comparatifs | | | | | | | | | |
|---|---|---|---|---|---|---|---|---|---|
| Comp.1 | Et | Me | 2 | OH | H | 0,92 | 15 | >5 x 10⁵ | >1,1x10⁶ |
| Comp 2 | Et | Me | 4 | OH | H | 2.3 | 30 | >0,5 x 10⁵ | >0,25 x 10⁵ |
| Comp.3 (Formule C) | Et | H | 2 | OH | H | 70 | | >2,5 x 10⁵ | >3,5x10³ |
| Comp. 4 (Formule A) | | | | | | 2 | | >3 x 10⁴ | >1,5 X10⁴ |

Comme on peut le voir sur le Tableau 1, les composés de la présente invention se sont révélés des antiviraux très actifs contre VIH-1, en particulier le Composé No. 3 ayant la Formule **I** dans lequel R¹ = -CHMe₂, R² = Me, R³ = -OH et R⁴ = H, c'est-à-dire le 1-(3-*N*¹-hydroxyuréidopropyloxyméthyl)-6-(3,5-diméthylbenzyl)-5-isopropyluracile, pour lequel on peut remarquer une activité 100.000 fois supérieure à celle de ses congénères ou de ses concurrents, et tout particulièrement le Composé No. 5 ayant la Formule **I** dans lequel R¹ = -CHMe₂, R² =Me, R³ = -OOCCMe₃ et R⁴ = -H, c'est-à-dire le 1-(3-*N*¹-pivaloyloxyuréido-propyloxyméthyl)-6-(3,5-diméthylbenzyl)-5-isopropyluracile, pour lequel on peut remarquer l'activité spectaculaire et imprévisible, à savoir une activité 1.000.000 fois supérieure à celle de ses congénères ou de ses concurrents.

La présente invention fournit donc un nouveau type d'acyclonucléosides comme agent antiviral, notamment agissant comme inhibiteur non nucléosidique de la transcriptase inverse du VIH-1, atoxique, et qui peut être actif à des concentrations picomolaires, voire femtomolaires.

En outre, lors de tests effectués sur l'un des composés de la présente invention, à savoir le Composé No. 2, il a été montré que ce composé présentait une synergie avec l'AZT (3'-azido-3'-désoxythymidine), la ddl (2',3'-didésoxyinosine) et la ddC (2',3'-didésoxycytidine), ce qui laisse entendre que le composé de la présente invention en général peut avantageusement être utilisé avec au moins l'AZT, la ddl et/ou la ddC.

Ainsi, le composé de la présente invention ou un sel pharmaceutiquement acceptable de celui-ci peut être utilisé comme ingrédient actif dans une composition pharmaceutique, et il peut être utilisé soit seul, soit en mélange avec d'autres ingrédients actifs, notamment dans une composition pharmaceutique destinée à être utilisée comme médicament dans le cadre d'une polythérapie.

La présente invention fournit donc également une composition pharmaceutique contenant en tant qu'ingrédient actif au moins un composé de la présente invention ou un sel pharmaceutiquement acceptable de celui-ci.

La quantité du composé de la présente invention ou de son sel pharmaceutiquement acceptable contenue dans la composition dépendra notamment du poids, de l'age et de l'état du patient ainsi que de l'efficacité du composé.

La composition pharmaceutique de la présente invention peut être sous une forme administrable par voie orale ou par voie systémique, et elle peut contenir tout support ou excipient pharmaceutiquement acceptable approprié.

Le composé de la présente invention contenu dans la composition pharmaceutique peut être avantageusement un composé dans lequel R³ ou R⁴ est un groupe -OR⁵.

Dans ce cas, le composé de la présente invention est un "prodrug" qui, après administration, sera transformé dans le corps en composé de la présente invention dans lequel R³ ou R⁴ est un groupe -OH.

Un tel composé où R³ ou R⁴ est un groupe -OR⁵ pourra notamment être avantageusement utilisé dans une formule retard.

Le composé de la présente invention dans lequel R⁵ est un groupe acyle est particulièrement avantageux car il est plus lipophile que son précurseur et le groupe N-OH est protégé contre l'oxydation. En outre, il peut être désacylé dans le sang à des vitesses contrôlables par la nature du groupe acyle.

L'utilisation de groupes R⁵ ramifiés, comme par exemple le groupe pivaloyle, est également avantageuse car elle permet de retarder l'hydrolyse du groupement ester par les estérases.

Grâce à la présente invention, il a été possible d'améliorer la fixation sur le récepteur, à savoir le site allostérique de la transcriptase inverse du VIH-1, ce qui se manifeste par une concentration inhibitrice plus faible, ce qui implique une meilleure sélectivité, un coût de préparation plus faible et une propension à sélectionner des mutants résistants plus faible.

Un autre avantage des acyclonucléosides à groupement *N*-hydroxyuréido de la présente invention est qu'ils peuvent être détectés par Résonance Paramagnétique Electronique (RPE) après oxydation en radicaux libres aminoxyles correspondants.

L'oxydation à l'air, éventuellement facilitée par une faible irradiation UV, conduit à une très faible concentration stationnaire d'espèces paramagnétiques qui, étant donné la grande sensibilité et de la grande sélectivité de la technique RPE, permet de suivre la molécule dans des milieux biologiques complexes.

Des tests de toxicité des Composés 3 et 5 de la présente invention ont encore été effectués sur des cellules de moelle osseuse selon le mode opératoire suivant.

Des cellules de moelle osseuse de souris Balb/c ont été cultivées à raison de 5 x 10⁵ cellules /ml dans des cultures de 1 ml dans du milieu RPMI 1640 supplémenté avec 1 mM de glutamine, 10 % de sérum de veau foetal, pénicilline et streptomycine (GIBCO, USA)

Les cellules ont été stimulées par addition de 1 ng/ml de Srem cell factor (c-kit ligand), d'IL3 et de GM-CSF (Pharmingen, USA) en présence de concentrations croissantes des Composés 3 et 5 de la présente invention allant de 1 x 10⁻¹² à 1 x 10⁻⁵ M.

Après 5 jours de culture à 37°C, 5% de CO₂, les cellules ont été récupérées.

La prolifération a été évaluée par numération des cellules vivantes en présence de bleu Trypan.

La différenciation des cellules a été évaluée en cytométrie de flux (Facs- Calibur, BECTON DICKINSON, USA) par double marquage des cellules avec des anticorps anti-CD38 couplés à la phycoérythrine (PE), anti-Gr1 couplés à la fluorescéine 5 (FI), anti Gr1-PE et anti CD11b-FI (Pharmingen, USA).

A toutes les concentrations testées, la prolifération cellulaire était identique à celle observée en l'absence du Composé 3 ou 5 de la présente invention.

De même, les proportions de lymphocytes B, de myélocytes, de neutrophiles et de macrophages induites par les cytokines étaient identiques en présence ou en absence du Composé 3 ou 5 de la présente invention, quelle que soit la concentration.

Ces résultats montrent clairement que les composés de la présente invention, et en particulier les Composés 3 et 5 de la présente invention n'ont pas d'effet toxique significatif sur des cellules de moelle osseuse de souris et ne gênent pas leur différenciation in vitro même à des concentrations 1 x 10⁶ fois supérieures à celles donnant une inhibition de la réplication du VIH dans des lymphocytes humains in vitro.

Les exemples suivants sont destinés à illustrer la présente invention. Cependant, ils ne doivent en aucun cas être considérés comme limitant l'étendue de la présente invention.

### EXEMPLES

Les produits de départ, les réactifs et les solvants utilisés dans les synthèses suivantes sont tous des produits commerciaux provenant de chez Fluka (Buchs, Switzerland), Merck (Darmstadt, Germany), ou Aldrich (Buchs, Switzerland), à moins qu'autre chose ne soit spécifié.

### Exemple 1

### Synthèse du 1-(3-N¹-hydroxyuréidopropyloxyméthyl)-6-(3,5-diméthylbenzyl)-5-isopropyluracile (Composé No. 3 de la présente invention)

(Composé de la présente invention de Formule **I,** dans lequel n=3, R¹ est un groupe isopropyle, chaque R² est un groupe méthyle, R³ est un groupe -OH et R⁴ est un atome d'hydrogène).

### A. Synthèse du 6-(3,5-diméthylbenzyl)-5-isopropyluracile

### (Composé de Formule VI selon le Schéma réactionnel 1 dans lequel R¹ est un groupe isopropyle et chaque R² est un groupe méthyle)

A une solution d'acide 2-bromo-3-méthylbutanoïque (3 g, 16,5 mmoles) dans de l'éthanol (200 ml), on a ajouté de l'acide sulfurique concentré (4 ml). Après 36 h d'ébullition à reflux, le milieu réactionnel, amené à température ambiante, a été neutralisé avec une solution aqueuse saturée de carbonate de sodium. Après distillation de l'éthanol, le milieu réactionnel a été extrait par du dichlorométhane (100 ml). La phase organique a ensuite été séchée sur du sulfate de magnésium et le solvant a été évaporé pour donner le 2-bromo-3-méthylbutanoate d'éthyle (1,91 g, 55 %).

Une suspension de poudre de zinc (31,5 g, 0,48 mole) dans du tétrahydrofurane (300 ml) a été portée à ébullition sous reflux, puis il a été ajouté quelques gouttes de 2-bromo-3-méthylbutanoate d'éthyle pour initier la réaction. Après 45 mn à reflux sous agitation magnétique, il a été ajouté du 3,5-diméthylphényléthanenitrile (13,2 g, 91 mmoles), puis goutte à goutte, le reste du 2-bromo-3-méthylbutanoate d'éthyle (au total 19,1 g, 91 mmoles). L'ébullition a été maintenue pendant 15 mn, le mélange a ensuite été refroidi, puis il a été ajouté à celui-ci du tétrahydrofurane (500 ml) et une solution aqueuse de carbonate de potassium à 50 % (100 ml). Après 45 mn d'agitation vigoureuse, la phase organique a été séparée par décantation et la phase aqueuse a été lavée avec du tétrahydrofuranne (2 x 100 ml). Les phases organiques rassemblées ont été traitées par une solution aqueuse à 10 % d'acide chlorhydrique (300 ml) pendant 45 mn. Après élimination du tétrahydrofurane par distillation sous pression réduite, le résidu a été repris par du dichlorométhane (300 ml), et la phase organique a été lavée par une solution saturée de monohydrogénocarbonate de sodium (100 ml), séchée sur du sulfate de magnésium et concentrée. La distillation du résidu (134 °C, 10⁻¹ mmHg) fournissait le 3-méthyl-2-(3,5-diméthylphénylacétyl)-butanoate d'éthyle (13,2 g, 52 %).

Du sodium métallique (23,8 g, 1.034 moles) a été mis en réaction avec de l'éthanol anhydre (500 ml). A la solution limpide obtenue, il a été ajouté de la thiourée (54,35 g, 714 mmoles) et du 3-méthyl-2(3,5-diméthylphénylacétyl)butanoate d'éthyle (13,14 g, 47,6 moles). Le milieu réactionnel a été maintenu à reflux pendant 6 h, puis concentré sous vide à 40,50 °C. Au résidu obtenu, il a été ajouté de l'acide chlorhydrique concentré (100 ml) puis la solution a été amenée à pH 4 par de l'acide acétique. Le 6-diméthylbenzyl-5-isopropyl-2-thiouracile obtenu a été dissous dans une solution aqueuse à 10 % d'acide chloroacétique (200 ml) et la solution a été maintenue à reflux pendant 24 h puis refroidie à température ambiante et le précipité obtenu a été séparé par filtration et lavé avec de l'éthanol froid puis de l'éther, puis séché sous vide à 40 °C pour fournir le 6-(3,5-diméthylbenzyl)-5-isopropyluracile (7 g, 54%).
Point de fusion : 213-214 °C.

### B. Synthèse du 1-acétoxy-3-acétoxyméthoxypropane

### (Composé de Formule VII selon le Schéma réactionnel 1, dans lequel n = 3)

A un mélange de 1,3-dioxane (3 ml, 0,035 mmole) et d'anhydride acétique (3.3 ml, 0,035 mmole) porté à 0 °C, il a été ajouté une goutte d'acide sulfurique concentré. Le mélange a ensuite été agité 14 h à 20 °C, additionné d'acétate de sodium (2 g) et filtré. Le résidu distillé sous vide (120 °C, 16 mmHg) fournissait le 1-acétoxy-3-acétoxyméthoxypropane (3,34 g, 50 %) sous forme d'une huile incolore. ¹H-RMN (200 MHz, CDCl₃) : δ 1,90 (quint., 2H, J=6Hz, CH₂-CH₂-CH₂),
2,04 (s, 3H, OAc), 2,09 (s, 3H, OAc), 3,69 (t, 2H, J=6Hz, OCH₂-CH₂-CH₂-OAc), 4,14 (t, 2H, J=6Hz, OCH₂CH₂CH₂OAc), 5,22 (s, 2H, AcOCH₂O).
IR (KBr) : νₘₐₓ 2994(ν_{C-H}), 1706 et 1682(ν_{C=O}), 1225, 1074 cm⁻¹.

### C. Synthèse du 1-(3-N¹-hydroxyuréidopropyloxyméthyl)-6-(3,5-diméthylbenzyl)-5-isopropyluracile (Composé No. 3 de la présente invention)

A une solution de 6-(3,5-diméthylbenzyl)-5-isopropyluracile (1,5 g, 5,5 mmoles) obtenu à l'étape A ci-dessus et de 1-acétoxy-3-acétoxyméthoxypropane (2,1 g, 11 mmoles) obtenu à l'étape B ci-dessus dans de l'éthanenitrile (60 ml), il a été ajouté de l'hexaneméthyldisilazane (1,78 g, 11 mmoles) et du chlorotriméthylsilane (1,2 g, 11 mmoles). Après 15 mn à 20 °C, il a été ajouté goutte à goutte une solution de chlorure d'étain^{IV} (2,87 g, 11 mmoles) dans de l'éthanenitrile (15 ml). Après 14 h d'agitation à 20 °C, il a été ajouté du dichlorométhane (100 ml) et une solution aqueuse saturée d'hydrogénocarbonate de sodium (50 ml). La phase organique a ensuite été lavée par une solution aqueuse saturée d'hydrogénocarbonate de sodium (30 ml) puis par une solution saturée de chlorure de sodium (30 ml), et séchée sur du sulfate de magnésium, puis concentrée. Le résidu obtenu a été soumis à une chromatographie sur colonne «flash» (éther de pétrole/acétate d'éthyle 1:1, R_{F} = 0,41) pour fournir le 1-acétoxypropyloxyméthyl)-6-(3,5-diméthylbenzyl)-5-isopropyluracile (1,73 g, 75 %) sous forme d'une huile visqueuse.

Ce dérivé acétylé (1,7 g, 4,05 mmoles) a été dissous dans du méthanol (40 ml) et il a été ajouté à celui-ci de la triéthylamine (5 ml) et de l'eau (5 ml). Après 48 h d'agitation à 20 °C, la réaction était terminée (CCM). Le milieu réactionnel a été concentré, les restes de produits volatils ont été éliminés par coévaporation avec du toluène et le résidu a été soumis à une chromatographie sur colonne « flash » (dichlorométhane/méthanol 99,5 :0.5) pour fournir le 1-(3-hydroxypropyloxyméthyl)-6-(3,5-diméthylbenzyl)-5-isopropyluracile (1,43 g, 93 %).
A une solution de cet alcool (1,43 g, 3,96 mmoles), de *N,O*-(diphénoxycarbonyl)-hydroxylamine (1,2 g, 4,36 mmoles) et de triphénylphosphine (1,25 g, 4,76 mmoles) dans du tétrahydrofurane (30 ml), il a été ajouté goutte à goutte à 0 °C une solution d'azodicarboxylate de diisopropyle (0,97 g, 4,76 mmoles) dans du tétrahydrofurane (5 ml). Le milieu réactionnel a été concentré puis soumis à une chromatographie sur colonne «flash» (méthanol/chloroforme 0,3:9,7) pour fournir le 6-(3,5-diméthylbenzyl)-1-[3-(*N*-phénoxycarbonyl-*N*-phénoxycarbonyloxyamino)propyloxyméthyl]-5-isopropyluracile (1,8 g, 79 %) sous la forme d'un solide,
Point de fusion : 59 - 61 °C.
1H-RMN (200 MHz, CDCl₃) : δ 1,23 (d, 6H, J=6,5Hz, CH-Me₂), 2,03 (quint, 2H, J=6Hz, CH₂-CH₂-CH₂), 2,29 (s, 6H, Me₂Ph), 2,82 (sept., 1 H, CHMe₂), 3,75 (t, 2H, OCH₂-CH₂-CH₂-N), 3,95 (t, 2H, NCH₂CH₂CH₂O), 4,09 (s, 2H, CH₂Ar), 5,12 (s, 2H, NCH₂O), 6,70 (s, 2H, H_{ortho-benzyle}), 6,90 (s, 1 H, H_{para-benzyle)}, 7,10-7,48 (m, 10, 2PhO), 8,1 (s I, 1H, NH).
IR (CH₂Cl₂) : νₘₐₓ 3382(ν_{N-H}), 3050-2870(ν_{C-H}), 1806, 1741, 1707 et 1682 (ν_{C=O}) cm⁻¹.
Analyse élémentaire pour C₃₄H₃₇N₃O₈ :
Calculée : C, 66,33; H, 6,06; N, 6,82.
Trouvée : C, 66,33; H, 6,12; N, 6,83.

Une solution de ce dernier composé (1,8 g, 3,13 mmoles) dans le méthanol saturé en ammoniac a été agitée à 20 °C jusqu'à disparition du produit de départ (réaction suivie par CCM). Le résidu a été soumis à une chromatographie sur colonne «flash» (méthanol/acétate d'éthyle 1:9) pour fournir le 1-(3-*N*¹-hydroxyuréidopropyloxyméthyl)-6-(3,5-diméthylbenzyl)-5-isopropyluracile désiré (0,74 g, 60 %) sous la forme d'un solide.
Point de fusion : 88 - 90°C.
1H-RMN (200 MHz, CDCl₃) : δ 1,29 (d, 6H, J=6,5Hz, CHMe₂), 1,89 (quint, 2H, J=6Hz, CH₂-CH₂-CH₂), 2,28 (s, 6H, Me₂Ph), 2,88 (sept., 1 H, CHMe₂), 3,62 (t, 2H, OCH₂-CH₂-CH₂-N), 3,68 (t, 2H, NCH₂CH₂CH₂O), 4,09 (s, 2H, CH₂Ar), 5,11 (s, 2H, NCH₂O), 6,70 (s, 2H, H_{ortho-benzyle}), 6,90 (s, 1 H, H_{para-benzyle}), 9,00 et 9,87 (2 s l, 3H, NH).
IR (CH₂Cl₂): νₘₐₓ 3530(ν_{O-H}), 3430, 3372 et 3190 (ν_{N-H}), 3000-2850 (ν_{c-H}), 1681,5 (ν_{C=O}) cm⁻¹.
Analyse élémentaire pour C₂₁H₃₀N₄Oₛ.1/2H₂O :
Calculée : C, 59,00; H, 7,31; N, 13,11.
Trouvée : C, 58,83; H, 7,15; N, 12,82.

### Exemple 2

### Synthèse du 1-(3-N¹-acétoxyuréidopropyloxyméthyl)-6-(3,5-diméthylbenzyl)-5-isopropyluracile (Composé No. 4 de la présente invention)

(Composé de la présente invention de Formule I, dans lequel n=3, R¹ est un groupe isopropyle, chaque R² est un groupe méthyle, R³ est un groupe -OR⁵ , où R⁵ est un groupe -CO-CH₃ et R⁴ est un atome d'hydrogène).

A une solution de 1-(3-*N*¹-hydroxyuréidopropyloxyméthyl)-6-(3,5-diméthylbenzyl)-5-isopropyluracile obtenu dans l'Exemple 1-C ci-dessus (85 mg, 0,2 mmole) dans du dichlorométhane (3,5 ml), il a été ajouté du DMAP (4-diméthylaminopyridine) (5 mg, 0,04 mmole), de la triéthylamine (45 µl, 0,3 mmole) et de l'anhydride acétique (25 µl, 0,24 mmole) et le mélange a été agité pendant 3 heures à 20°C. Ensuite, il a été ajouté du dichlorométhane (5 ml) et une solution aqueuse saturée d'hydrogénocarbonate de sodium (5 ml). Le milieu réactionnel a ensuite été extrait par du dichlorométhane (50 ml) et la phase organique a été lavée par de l'eau (10 ml) puis par une solution saturée de chlorure de sodium (10 ml) et séchée (sulfate de sodium). La phase organique a ensuite été concentrée et soumise à une chromatographie "flash" sur une colonne de gel de silice (dichlorométhane/méthanol 9 : 1) pour fournir 70 mg (75 %) du 1-(3-*N*¹-acétoxyuréidopropyloxyméthyl)-6-(3,5-diméthylbenzyl)-5-isopropyluracile désiré sous la forme d'un solide.
Point de fusion : 77 - 79°C.
1 H-RMN (200 MHz, CDCl₃) : δ 1,31 (d, 6H, J=7,OHz, CHMe₂), 1,84 (quint, 2H, J=6,5Hz, CH₂-CH₂-CH₂), 2,22 (s, 3H, OAc), 2,31 (s, 6H, Me₂Ph), 2,86 (sept., 1 H, CHMe₂), 3,67 (t, 2H, OCH₂-CH₂-CH₂-N), 3,75 (t, 2H, NCH₂CH₂CH₂O), 4,10 (s, 2H, CH₂Ar), 5,10 (s, 2H, NCH₂O), 6,72 (s, 2H, H_{ortho-benzyle}), 6,91 (s, 1H, H_{para-benzyle}), 8,65 (s l, 1H, NH).
IR (CH₂Cl₂) : νₘₐₓ 3531, 3420, 3372, 3195 (ν_{N-H}), 3055-2871 (ν_{C-H}), 1795 (ν_{C=O} Ac), 1784, 1683 (ν_{C=O} CON) cm⁻¹.
SM (m/z (%)) 444 (2, M⁺ - NH₂), 385 (16, M⁺ - NH₂ - AcO), 302 (1, BH⁺), 301 (7; B⁺), 287 (2, BH⁺ - Me), 286 (B⁺ - Me).
Analyse élémentaire pour C₂₃H₃₂N₄O₆.1/2H₂O (469,61) :
Calculée : C, 58,83; H, 7,08; N, 11,93.
Trouvée : C, 58,92; H, 6,98; N, 11,83.

### Exemple 3

### Synthèse du 1-(3-N¹-pivaloyloxyuréidopropyloxyméthyl)-6-(3,5-diméthylbenzyl)-5-isopropyluracile (Composé No. 5 de la présente invention)

(Composé de la présente invention de Formule **I,** dans lequel n=3, R¹ est un groupe isopropyle, chaque R² est un groupe méthyle, R³ est un groupe pivaloyloxy (Me₃CCOO-) et R⁴ est un atome d'hydrogène).

A une solution de 1-(3-*N*¹-hydroxyuréidopropyloxyméthyl)-6-(3,5-diméthylbenzyl)-5-isopropyluracile obtenu dans l'Exemple 1-C ci-dessus (134 mg, 0,32 mmole) dans de la pyridine (1,5 ml), il a été ajouté 46 mg (0,38 mmole) de chlorure de pivaloyle. Après 14 h d'agitation à 24 °C, la pyridine a été éliminée par coévaporation avec du toluène (2 x 3ml) et le résidu a été repris par du dichlorométhane (10 ml). La phase organique a ensuite été lavée par une solution aqueuse saturée d'hydrogénocarbonate de sodium (10 ml). La phase aqueuse a été extraite par du dichlorométhane (3 x 10 ml) et les phases organiques, rassemblées, lavées par une solution aqueuse d'hydrogénocarbonate de sodium (10 mL), une solution aqueuse saturée de chlorure de sodium (10 mL), séchées (sulfate de sodium) abandonnent par distillation du solvant 180 mg de 1-(3-*N*¹-pivaloyloxyuréidopropyloxyméthyl)-6-(3,5-diméthylbenzyl)-5-isopropyluracile brut qui a été soumis à une chromatographie "flash" sur une colonne de gel de silice (dichlorométhane/méthanol 24:1) pour fournir 127 mg (78%) du 1-(3-*N*¹-pivaloyloxyuréidopropyloxyméthyl)-6-(3,5-diméthylbenzyl)-5-isopropyluracile désiré sous forme d'un solide.
Point de fusion 132-133°C;
R_{F} 0,22 (dichlorométhane/méthanol 24:1).
UV (MeOH) nm (ε): 210 (17500) et 268 (9420).
¹H RMN (200 MHz, CDCl₃): δ 1,27 (d, 6 H, J = 6,5 Hz, CHMe₂), 1,29 (s, 9 H, CMe₃), 1,80 (*quint.,* 2 H, J = 6 Hz, CH₂-CH₂-CH₂), 2,28 (s, 6 H, Me₂Ph), 2,86 (*sept,* 1 H, CHMe₂), 3,62 (t, 2 H, OCH₂CH₂CH₂N), 3,72 (t, 2 H; OCH2CH₂CH₂N), 4,08 (s, 2 H, CH₂Ar), 5,09 (s, 2 H, NCH₂O), 6,69 (S, 2 H, H_{*ortho*-benzyle}), 6,89 (s, 1 H, H_{*para*-benzyle)}, 8,66 (s *él,* 1 H NH).
¹³C RMN (50 MHz, CDCl₃) : δ 20,4 (Me₂Ar), 21,3 (Me₂CH), 26,8 (NCH₂CH₂CH₂-O), 27,0 (Me₃C), 28,4 (Me₂CH), 33,3 (CH₂Ar), 38,5 (Me₃C), 46,6 (NCH₂-CH₂CH₂), 66,4 (OCH₂CH₂CH₂N), 73,1 (NCH₂O), 119,6 (C-5), 125,0 (C*ₒᵣₜₕₒ*-Ar), 128,8 (C*ₚₐᵣₐ*-Ar), 135,1 (C*ᵢₚₛₒ*-Ar), 138,8 (C*_{méta}*-Ar), 148,8 (C-2), 151,8 (C-6), 158,8 (CONH₂), 162,3 (C-4), et 175,7 (COCMe₃).
IR (CH₂Cl₂): νₘₐₓ 3535 (ν_{N-H}), 3430 et 3386 (ν_{NH2}), 3052 (ν_{C-H} asymétrique), 1779 (ν_{C=O} ester), 1705, 1684 (ν_{C=O} CON) cm⁻¹.
SM m/z (%): 355 (4, B-CH₂-OCH₂CH₂CN⁺), 301 (2, B-CH₂O⁺), 285 (3, B-CH₂⁺), 273 (13, BH₂⁺), 272 (62, BH⁺), 271 (5, B⁺), 257 (100, BH⁺ - Me).
Analyse élémentaire pour C₂₆H₃₈N₄O₆ (502,62), 0,5 CH₂Cl₂):
Calculée : C, 58,39; H, 7,21; N, 10,28.
Trouvée : C, 58,14; H, 7,20; N, 10,13.

### Exemple 4

### Synthèse du 1-(3-N³-hydroxyuréidopropyloxyméthyl)-6-(3,5-diméthylbenzyl)-5-éthyluracile (Composé No. 1 de la présente invention)

(Composé de Formule I de la présente invention dans lequel n= 3, R¹ est un groupe éthyle, chaque R² est un groupe méthyle, R³ est un atome d'hydrogène et R⁴ est un groupe OH)

### A. Synthèse du 5-éthyl-1-(3-hydroxypropopyloxyméthyl)-6-(3,6-diméthylbenzyle)uracile

### (Composé de Formule IX selon le Schéma réactionnel 1 dans lequel R¹ est un groupe éthyle et chaque R² est un groupe méthyle)

A une suspension de 5-éthyl-6-(3,5-diméthylbenzyl)uracile (645 mg, 5 mmoles) (préparé comme décrit pour la préparation du 6-(3,5-diméthylbenzyl)-5-isopropyluracile dans l'Exemple 1-A ci-dessus mais en remplaçant l'acide 2-bromo-3-méthylbutanoïque par l'acide 2-bromobutanoïque) et de 1-acétoxy-3-acétoxy-méthoxypropane (préparé comme décrit dans l'Exemple 1-B ci-dessus) (950 mg, 5 mmoles) dans de l'éthanenitrile (25 ml), il a été ajouté de l'hexaméthyldisilazane (810 mg, 5 mmoles) et du chlorotriméthylsilane (545 mg, 5 mmoles). Après 10 minutes d'agitation à 20 °C, il a été ajouté goutte à goutte, en 10 minutes, une solution de chlorure d'étain^{IV} (782 mg, 2,4 mmoles) dans de l'éthanenitrile (7 ml). Après 14 h d'agitation, il a été ajouté au milieu réactionnel, du dichlorométhane (70 ml) et une solution aqueuse saturée d'hydrogénocarbonate de sodium (70 ml). La phase organique a été séparée par décantation et la phase aqueuse a été extraite par du dichlorométhane (3 x 50 ml). Les phases organiques rassemblées ont été lavées par une solution saturée d'hydrogénocarbonate de sodium (25 ml), de l'eau (25 ml) et une solution aqueuse saturée de chlorure de sodium (25 ml). La phase organique a été séchée sur du sulfate de sodium, concentrée, et soumise à une chromatographie sur colonne «flash» de gel de silice (acétate d'éthyle/éther de pétrole 1:1) pour fournir 0,85 g (87 %) de 5-éthyl-1-(3-acétoxypropyloxyméthyl)-6-(3,5-diméthylbenzyl)uracile.

| Analyse élémentaire pour C₂₁H₂₈N₂O₅ (388,47) : | |
|---|---|
| Calculée : | C, 44,93; H, 7,26; N, 7,21. |
| Trouvée : | C, 44,76; H, 7,39; N, 7,11. |

Ce dernier composé a ensuite été agité pendant 34 h dans un mélange de triéthylamine-méthanol-eau 1:8:1 (80 ml) à 20 °C pour fournir, après purification par chromatographie «flash» sur colonne de gel de silice (dichlorométhane/méthanol 19:1), 0,57 g (76 %) de 5-éthyl-1-(3-hydroxypropyloxyméthyl)-6-(3,5-diméthylbenzyl)uracile.

| Analyse élémentaire pour C₁₉H₂₆N₂O₄ (346,43) : | |
|---|---|
| Calculée : | C, 65,88; H, 7,56; N, 8,09. |
| Trouvée : | C, 65,42; H, 7,46; N, 7,94. |

### B. Synthèse du 1-(3-N³-hydroxyuréidopropyloxyméthyl)-6-(3,5-diméthylbenzyl)-5-éthyluracile (Composé No. 1 de la présente invention)

Le 5-éthyl-1-(3-hydroxypropyloxyméthyl)-6-(3,5-diméthyl-benzyl)uracile obtenu à l'étape A ci-dessus (210 mg, 0,6 mmole), du phthalimide (110 mg, 0,72 mmole) et de la triphénylphosphine (0,19 g, 0,72 mmole) ont été dissous dans du tétrahydrofurane et la solution a été refroidie à 0 °C. Il a été ensuite ajouté à celle-ci goutte à goutte une solution d'azodicarboxylate d'isopropyle (145 mg, 0,72 mmole) dans du tétrahydrofurane (2 ml). Le milieu réactionnel a ensuite été concentré et soumis à une chromatographie «flash» sur colonne de gel de silice (dichlorométhane/méthanol 49:1) et le produit obtenu a été purifié par une nouvelle chromatographie «flash» sur colonne de gel de silice (acétate d'éthyle/éther de pétrole 1:1) pour fournir du 5-éthyl-6-(3,5-diméthylbenzyl)-1-(3-phthalimidopropyloxy-méthyl)uracile (0,23 g, 79 %).

Ce dernier composé a été ensuite hydrazinolysé par 2 h d'agitation à 20 °C dans une solution éthanolique (5 ml) d'hydrate d'hydrazine (240 mg) et purifié par chromatographie sur gel de silice (dichlorométhane/méthanol/méthanol saturé d'ammoniac 8:2:0,25) pour fournir du 1-(3-aminopropyloxyméthyl)-5-éthyl-6-(3,5-diméthylbenzyl)uracile (0,10 g, 59 %).

Ce dernier composé (100 mg, 0,29 mmole), en solution dans du dichlorométhane (3 ml), a été ajouté à une solution de carbonyldiimidazole (66 mg, 0,41 mmole) dans du dichlorométhane (6 ml) dans laquelle il avait été ajouté goutte à goutte une solution d'*O*-*ter*-butyldiméthylsilylhydroxylamine (60 mg, 0,41 mmole) dans du dichlorométhane (1 ml) et qu'on avait agitée 20 mn à 20 °C. Après deux chromatographies sur colonne «flash» de gel de silice (dichlorométhane/méthanol 19:1), le produit a été O-désilylé et il a été obtenu 60 mg (52 %) de 1-(3-*N*³-hydroxyuréidopropyloxyméthyl)-6-(3,5-diméthylbenzyl)-5-éthyluracile désiré sous la forme d'une huile.
¹H-RMN (200 MHz, CDCl₃, 40°C) : δ 1,07 (t, 3H, J=7,0Hz, CH₂CH₃), 1,82 (quint, 2H, J=6Hz, CH₂-CH₂-CH₂), 2,30 (s, 6H, Me₂Ph), ca 2,5 (s I, 3H, NH), 2,51 (q, 2H, CH₂CH₃), 3,40 (t, 2H, NCH₂-CH₂-CH₂-O), 3,70 (t, 2H, OCH₂CH₂CH₂N), 4,09 (s, 2H, CH₂Ar), 5,13 (s, 2H, NCH₂O), 6,72 (s, 2H, H_{ortho-benzyle}), 6,92 (s, 1H, H_{para-benzyle}).
IR (KBr) : νₘₐₓ 3401 (ν_{O-H}), 3291, 3186 et 3058 (ν_{N-H}), 1691 (vc=o) cm⁻¹.

| Analyse élémentaire pour C₂₀H₂₈N₄O₅.1/3H₂O: | |
|---|---|
| Calculée : | C, 58,52; H, 7,04; N, 13,65. |
| Trouvée : | C, 58,63; H, 6,99; N, 13,44. |

### Exemple 5

### Synthèse du 1-(3-N¹-hydroxyuréidopropyloxyméthyl)-6-(3,5-diméthylbenzyl)-5-éthyluracile (Composé No. 2 de la présente invention)

### (Composé de Formule 1 de la présente invention dans lequel n = 3, R¹ = éthyle, R² = méthyle, R³ = OH, R⁴ = H).

A une solution de 5-éthyl-1-(3-hydroxypropyloxyméthyl)-6-(3,5-diméthylbenzyl)-; uracile (préparé comme décrit dans l'Exemple 4-A ci-dessus) (190 mg, 0,548 mmole), de *N*,O-bis(phénoxycarbonyl)hydroxylamine (165 mg, 0,603 mmole) et de triphénylphosphine (288 mg, 1,09 mmoles) dans du tétrahydrofurane (5 ml), il a été ajouté goutte à goutte à 0 °C une solution de DIAD (diisopropylazodicarboxylate) (222 mg, 1,09 mmoles) dans du tétrahydrofurane (1 ml). Le milieu réactionnel a alors été ramené à 20 °C, concentré, et soumis à une chromatographie sur colonne de gel de silice (acétate d'éthyle/éther 1:9) puis à une chromatographie sur colonne «flash» de gel de silice (méthanol/chloroforme 3:97) pour conduire à 250 mg de composé intermédiaire 6-(3,5-diméthylbenzyl)-1-[3-(*N*-phénoxycarbonyl-*N-*phénoxycarbonyloxyamino)propyloxyméthyl]-5-éthyluracile.

Ce composé intermédiaire a été immédiatement dissous dans une solution méthanolique saturée d'ammoniac (5 ml) et le milieu réactionnel a été agité à 20 °C pendant 24 h. A ce moment, le composé intermédiaire avait intégralement réagi (chromatographie sur couche mince) et le milieu réactionnel, concentré, a été soumis à une chromatographie sur colonne «flash» sur gel de silice (méthanol/acétate d'éthyle 1:9) pour fournir le 1-(3-*N¹*-hydroxypropyloxyméthyl)-6-(3,5-diméthylbenzyl)-5-éthyluracile désiré (40 mg, 24 %) sous forme d'une huile visqueuse qui cristallise spontanément en quelques heures à 20 °C.
Point de fusion : 165-167 °C ;
R_{F} 0,37 (méthanol/acétate d'éthyle 1:9).
¹H RMN (200 MHz, CD₃OD, 40 °C) : δ 0,90 (*t*, 3 H, J = 7,5 Hz, CH₂CH₃), 1,85 (*quint.,* 2 H, J = 6,25 Hz, NCH₂CH₂CH₂O), 2,25 (s, 6 H, Me₂Ph), 2,40 (*q*, 2 H, CH₂CH₃), 3,50 (*t*, 2 H, NCH₂CH₂CH₂O), 3,85 (*t*, 2 H, NCH₂CH₂CH₂O), 4,05 (s, 2 H, CH₂Ph), 5,07 (s, 2 H, OCH₂N), 6,65 (s *él.,* 2 H, H*ₒᵣₜₕₒ*-benzyle), 6,85 (*s*. *él.,* 1 H, H*ₚₐᵣₐ*-benzyle.
IR (KBr) : 3477, 3368 et 3159 (ν_{NH} et ν_{OH}), 1695 et 1650 (ν_{C=O}) cm⁻¹.
SM *(m*/*z* (%)) 359 (1, M^{.+}- NH₂COH), 341 (7, M^{.+}- NH₂COH - H₂O), 271 (41, M^{.+}- NH₂CONOH(CH₂)₃O), 258 (78, M^{.+}- NH₂CONOH(CH₂)₃OCH).

| Analyse élémentaire pour C₂₀H₂₈N₄O₅.1/3H₂O : | |
|---|---|
| Calculée : | C, 58,52; H, 7,04; N, 13,64. |
| Trouvée : | C, 58,55; H, 6,95; N, 13,49. |

### Exemple 6

### Synthèse du 1-(3-N¹-benzoyloxyuréidopropyloxyméthyl)-6-(3,5-diméthylbenzyl)-5-isopropyluracile (Composé No. 6 de la présente invention)

### (Composé de Formule I de la présente invention dans lequel n = 3, R¹ = isopropyle, R² = méthyle, R³ = benzoyloxy, R⁴ = H).

A une solution de 1-(3-*N*¹-hydroxyuréidopropyloxyméthyl)-6-(3,5-diméthylbenzyl)-5-isopropyluracile obtenu dans l'Exemple 1-C ci-dessus (100 mg, 0,24 mmoles) dans de la pyridine (5 ml), il a été ajouté 30 µl (0,26 mmoles) de chlorure de benzoyle. Après 14 heures d'agitation à 24°C, la pyridine a été éliminée par coévaporation avec du toluène (2 x 10 ml) et le résidu purifié par chromatographie sur une colonne de gel de silice (éther de pétrole/acétone 3 : 2) pour fournir 110 mg (88 %) du 1-(3-*N*¹-benzoyloxyuréidopropyloxyméthyl)-6-(3,5-diméthylbenzyl)-5-isopropyluracile désiré sous forme d'un solide.
Point de fusion : 160-161 °C ;
R_{F} 0,16 (éther de pétrole/acétone 3:2).
¹H RMN (200 MHz, CDCl₃): δ 1,28 (d, 6 H, J = 7,0 Hz, CHMe₂), 1,92 (*quint*., 2 H, J = 6,0 Hz, CH₂CH₂CH₂), 2,30 (s, 6 H, Me₂Ph), 2,84 (*sept.,* 1 H, Me₂CH), 3,70 (t, 2 H, NCH₂CH₂CH₂O), 3,87 (t, 2 H, OCH₂CH₂CH₂N), 4,08 (s, 2 H, CH₂Ar), 5,10 (s, 2 H, NCH₂O), 5,22 (*s élargi,* 2 H, NH₂), 6,70 (s, 2 H, H_{ortho-benzyle}), 6,90 (s, 1 H, Hpₐᵣₐ-_{benz}yₗₑ), 7,52 (*dt,* 2 H, Jₒᵣₜₕₒ = 9,0 HZ, Jₚₐᵣₐ = 0,5 Hz, H_{méta-benzoyle}), 7,70 (*dt,* 1 H, Jₘₑₜₐ = 3,5 Hz, H_{para-benzoyle}), 8,10 (*dd,* 2 H, H_{ortho-benzoyle}), 8,98 (*s élargi,* 1 H, NH).
IR (KBr) : νₘₐₓ 3431, 3363, 3205 (ν_{N-H}), 3028-2871 (ν_{C-H}), 1763 (ν_{C=O} benzoyle), 1680 (ν_{C=O} NC=O) cm⁻¹.
SM (*m*/*z* (%)) 398 (0,5, M^{+.} - OBz), 355 (2, M^{+.} - OBz-CONH₂), 272 (60, BH⁺), 257 (100, BH⁺-Me), 122 (86, OBz), 105 (95, Bz).

| Analyse élémentaire pour C₂₈H₃₄N₄O₆.(522,61) : | |
|---|---|
| Calculée : | C, 64,35; H, 6,56; N, 10,72. |
| Trouvée : | C, 64,26; H, 6,63; N, 10,56. |

### Exemple 7

### Synthèse du 1-(2-N¹-hydroxyuréidoéthoxyméthyl)-6-(3,5-diméthylbenzyl)-5-éthyluracile (Composé comparatif Comp. 1)

### (Composé de Formule 1 comparatif dans lequel n = 2, R¹ = éthyle, R² = méthyle, R³ = OH, R⁴ = H).

### A. Synthèse du 1-(2-acétoxyéthoxyméthyl)-6-(3,5-diméthylbenzyl)-5-éthyluracile

Du 6-(3,5-diméthylbenzyl)-5-éthyluracile (voir Exemple 4-A ci-dessus) (516 mg, 2 mmoles) et du 1-acétoxy-2-acétoxyméthoxyéthane [A. Rosowski, S. H. Kim. M. Wick, J. Med. Chem. (1981) 24, 1177-81]) (704 mg, 4 mmoles) ont été mis en suspension, sous atmosphère d'azote, dans de l'acétonitrile (20 ml). On a ajouté de l'HMDS (hexaméthyldisilazane) (646 mg, 4 mmoles) et du TMSCI (chlorotriméthylsilane) (435 mg, 4 mmoles) et on a agité pendant 10 mn. On a ajouté alors goutte à goutte en 10 mn, une solution de chlorure d'étain^{IV} (625 mg, 2,4 mmoles) dans de l'acétonitrile (5 ml). Après 14 h d'agitation, on a ajouté du dichlorométhane (50 ml) et une solution aqueuse saturée d'hydrogénocarbonate de sodium (50 ml). On a extrait par du dichlorométhane (3 x 50 ml). Les phases organiques, rassemblées, ont été lavées par une solution aqueuse saturée d'hydrogénocarbonate de sodium (25 ml), par de l'eau (25 ml), puis par une solution aqueuse saturée de chlorure de sodium. La phase organique a été séchée (sulfate de sodium), concentrée, puis a été soumise à une chromatographie sur colonne «flash» (éther de pétrole/acétate d'éthyle 3:2) pour fournir le 1-(2-acétoxyéthoxyméthyl-6-(3,5-diméthylbenzyl)-5-éthyluracile (690 mg, 92 %) sous forme d'un solide blanc.
Point de fusion : 126,0-127,0 °C
R_{F} 0,28 (hexane/acétate d'éthyle 3:2).
¹H RMN (CDCl₃, 200 MHz) : δ 1,10 (*t,* 3 H, J = 7,25 Hz, CH₂CH₃), 2,08 (*s*, 3 H, Ac), 2,30 (s, 6 H, Me₂Ph), 2,69 (*q,* 2 H, CH₂CH₃), 3,80 (*m,* 2 H, AcOCH₂**CH₂**O), 4,08 (s, 2 H, CH₂Ph), 4,20 (*m,* 2 H, AcOCH₂), 5,18 (*s*, 2 H, OCH₂N), 6,71 (s, 2 H, H*ₒᵣₜₕₒ*-benzyle), 6,90 (s, 1 H, H*ₚₐᵣₐ*-benzyle), 8,25 (*s él.* 1 H, NH).
IR (KBr) : 3030 (ν_{C-H} arom.), 2950 (ν_{C-H} aliphatique), 1740 et 1705 (ν_{C=O}) cm⁻¹.
UV (méthanol) νₘₐₓ (v) 266 nm (8180).
SM (*m*/*z* (%)) 374 (4, M^{.+}), 255 (16, M^{.+} - AcOCH₂CH₂O), 119 (11, M^{.+} - base), 87 (100, AcOCH₂CH₂⁺).

| Analyse élémentaire pour C₂₀H₂₆N₂O₅ (374,44) : | |
|---|---|
| Calculée : | C, 64,16; H, 7,00; N, 7,48. |
| Trouvée : | C, 63,88; H, 7,10; N, 7,37. |

### B. Synthèse du 6-(3,5-diméthylbenzyl)-5-éthyl-1-(2-hydroxyéthoxyméthyl)uracile

Une solution de 1-(2-acétoxyéthoxyméthyl)-6-(3,5-diméthylbenzyl)-5-éthyluracile (690 mg, 1,9 mmoles) dans un mélange méthanol/triéthylamine/eau 8:1:1 a été agitée 20 h à 20 °C. Les solvants ont été éliminés par évaporation sous vide puis par coévaporation avec du toluène. Le solide obtenu, recristallisé (méthanol) fournissait 390 mg de 6-(3,5-diméthylbenzyl)-5-éthyl-1-(2-hydroxyéthoxyméthyl)uracile. Les liqueurs-mères de cristallisation concentrées, soumises à une chromatographie sur colonne «flash» sur gel de silice (dichlorométhane/méthanol 19:1) fournissaient un échantillon supplémentaire de 80 mg. Rendement total 470 mg (77 %) d'un solide blanc.
Point de fusion : 178,0-179,0 °C ;
R_{F} 0,12 (éther de pétrole/acétate d'éthyle 9:1)
¹H RMN (CD₃OD, 200 MHz) : δ 1,01 *(t.* 3 H, J = 7,5 Hz, CH_{2C}H₃), 2,25 (s, 6 H, Me₂Ph), 2,42 (q, 2 H, CH₂CH₃), 3,60 (m, 4 H, CH₂CH₂), 4,12 (s, 2 H, CH₂Ph), 5,14 (s, 2 H, OCH₂N), 6,78 (s *él.* 2 H, H*ₒᵣₜₕₒ*-benzyle), 6,90 (s *él.,* 1 H, H-*ₚₐᵣₐ*-benzyle).
IR (KBr) : 3380 (ν_{O-H}), 3020 (ν_{C-H} arom.), 1708 (ν_{C=O}) cm⁻¹.
UV (méthanol) νₘₐₓ (v) 207 (41500), 268 (15400).
SM (*m*/*z* (%)) : 332 (2, M^{.+}), 258 (100, B⁺).

| Analyse élémentaire pour C₁₈H₂₄N₂O₄ (332,40) : | |
|---|---|
| Calculée : | C, 65,04; H, 7,28; N, 8,43. |
| Trouvée : | C, 64,93 ; H, 7,30 ; N, 8.36). |

### C. Synthèse du 1-(2-N¹-hydroxyuréidoéthoxyméthyl)-6-(3,5-diméthylbenzyl)-5-éthyluracile (Composé comparatif Comp. 1)

A une solution de 6-(3,5-diméthylbenzyl)-5-éthyl-1-(2-hydroxyéthoxyméthyl)uracile (0,2 g, 0,6 mmole), de *N*,*O-bis*(phénoxycarbonyl)hydroxylamine (0,18 g, 0,66 mmole) et de triphénylphosphine (0,19 g, 0,66 mmole) dans du tétrahydrofurane (6 ml), il a été ajouté goutte à goutte à 0 °C du DIAD (diisopropylazodicarboxylate) (145 mg, 0,71 mmole). Le milieu réactionnel, concentré, a été soumis à deux chromatographies sur colonne «flash» de gel de silice successivement, la première utilisant comme éluant le mélange dichlorométhane/méthanoi 49:1 et la seconde, le mélange acétate d'éthyle/éther de pétrole 2:3. On a obtenu ainsi du 6-(2,3-diméthylbenzyl)-5-éthyl-1-(2-N,O-bis(phénoxycarbonyl)hydroxyaminoéthoxyméthyl) uracile (0,30 g, 85 %) dont 0,15 g (0,25 mmole) a été dissous dans une solution méthanolique saturée d'ammoniac (10 ml) et agité 24 h à 10 °C. Le milieu réactionnel concentré, soumis à une chromatographie sur colonne «flash» de gel de silice (dichlorométhane/méthanol 19:1) fournissait une huile qui, reprise par de l'éther fournissait le 1-(2-*N*¹-hydroxyuréidoéthoxyméthyl)-6-(3,5-diméthylbenzyl)-5-éthyl-uracile (36,6 mg, 36 %) sous forme d'un solide blanc.
Point de fusion : 147 - 148°C
R_{F} 0,18 (dichlorométhane/méthanol 9:1)
¹H RMN (CDCl₃, 200 MHz) : δ 1,09 (*t*, 3 H, J = 7,5 Hz, CH₂CH₃), 2,29 (*s*, 6 H, Me₂Ph), 2,48 (*q*, 2 H, CH₂CH₃), 3,22-3,90 (*m*, 4 H, CH₂CH₂), 4,07 (*s*, 2 H, CH₂Ph), 5,10 (*s*, 2 H, OCH₂N), 6,70 (*s*, 2 H, H_{*ortho*-}benzyle), 6,90 (*s*, 1 H, H*ₚₐᵣₐ*-benzyle), 9,76 (*s él.,* 1 H, NH).
IR (KBr) : 3498, 3235 et 3190 (ν_{OH} et ν_{NH}), 1703, 1699 et 1688 (ν_{C=O}) cm⁻¹.
SM (m/z (%)) 345 (1, M^{.+} - NH₂CHO), 327 (6, M^{.+} - NH₂COH - H₂O), 271 (100, M^{.+} - NH₂CONOHCH₂CH₂O), 258 (73, B⁺).

| Analyse élémentaire pour C₁₉H₂₆N₄O₅.1/2H₂O (339,45): | |
|---|---|
| Calculée : | C, 57,13; H, 6,81; N, 14,03. |
| Trouvée . | C, 56,85; H, 6,63; N, 13,57. |

### Exemple 8

### Synthèse du 1-(4-N¹-hydroxyuréidobutyloxyméthyl)-6-(3,5-diméthylbenzyl)-5-éthyluracile (Composé comparatif Comp. 2)

### (Composé de Formule I comparatif dans lequel n = 4, R¹ = éthyle, R² = méthyle, R³ = OH, R⁴= H).

### A. Synthèse du benzoate de 4-hydroxybutyle

A une solution de 1,4-butanediol (18 ml, 0,2 mmole) dans de la pyridine (16 ml), on a ajouté lentement à 0 °C, sous atmosphère d'azote, du chlorure de benzoyle (1 ml), 0,1 mmole). Après 4 h d'agitation à 0 °C, le milieu réactionnel a été concentré et la pyridine a été éliminée par co-distillation avec du toluène. Le résidu été extrait à l'éther (3 x 20 ml) et la phase organique a été séchée (sulfate de magnésium), concentrée, et soumise à une chromatographie sur colonne «flash» de gel de silice (éther de pétrole/acétate d'éthyle 3:2) pour fournir le benzoate de 4-hydroxybutyle (9,9 g, 55 %) sous forme d'une huile incolore.
R_{F} 0,36 (éther de pétrole/acétate d'éthyle 1:1).
¹H RMN (CDCl₃, 200 MHz) : δ 1,79 (*m*, 4 H, CH₂CH₂CH₂CH₂), 3,60 (*t,* 2 H, J = 7.0 Hz, CH₂OH), 4,34 (*t*, 2 H, J = 8.0 Hz, PhCOOCH₂), 7, 45-8,02 (*m*, 5 H, Ph).

### B. Synthèse du 1-(4-benzoyloxybutyloxyméthyl)-6-(3,5-diméthylbenzyl)-5-éthyluracile

A une solution de benzoate de 4-hydroxybutyle (1 g, 5,2 mmoles) dans du dichlorométhane (18 ml), il a été ajouté du paraformaldéhyde (100 mg, 5,2 mmoles). Dans le mélange, porté à 0 °C, on a fait barboter pendant 2 h de l'acide chlorhydrique. Après ce temps, le récipient a été bouché et on a agité à 4 °C pendant 16 h. Le mélange réactionnel a été dilué par du dichlorométhane (10 ml), séché (sulfate de magnésium), puis concentré. Cette solution a été ajoutée à une suspension de 6-(3,5-diméthylbenzyl)5-éthyluracile (obtenu comme décrit dans l'Exemple 3) (400 mg, 1,55 mmoles) et de *N*,O-bis(triméthylsilyl)acétamide (1,1 ml, 4,4 mmoles) dans du dichlorométhane (10 ml) préalablement agitée 30 mn à 20 °C puis additionnée d'iodure de tétrabutylammonium (20 mg, 0,05 mmole). Le mélange a été chauffé à reflux pendant 16 h puis ramené à 20 °C. Il a ensuite été versé dans une solution aqueuse saturée d'hydrogénocarbonate de sodium maintenue à 0 °C et le mélange a été agité pendant 30 mn. La phase organique, décantée, a été lavée avec une solution aqueuse saturée de chlorure de sodium (15 ml), séchée (sulfate de magnésium), concentrée et soumise à une chromatographie sur colonne «flash» de gel de silice (éther de pétrole/acétate d'éthyle 7:3) qui fournissait le 1-(4-benzoyloxybutyloxyméthyl)-6-(3,5-diméthylbenzyl)-5-éthyluracile (460 mg, 60 %) sous forme d'une huile.
R_{F} 0,71 (dichlorométhane/méthanol 19:1).
¹H RMN (CDCl₃, 200 MHz) : δ 1,08 (t, 3 H, J = 8,0 Hz, CH₂CH₃), 1,78 (m, 4 H, CH₂CH₂CH₂CH₂), 2,29 (s, 6 H, Me₂Ph), 2,48 (q, 2 H, CH₂CH₃), 3,61 (t, 2 H, J = 5,5 Hz, (CH₂)₃CH₂OCH₂), 4,09 (s, 2 H, CH₂Ph), 4,34 (t, 2 H, J = 6,0 Hz, PhCOOCH₂), 5,11 (s, 2 H, OCH₂N), 6,70 (s *él.,* 2 H, H*ₒᵣₜₕₒ*-benzyle), 6,91 (s, 1 H, H*ₚₐᵣₐ*-benzyle), 7,40-8,09 (m, 5 H, PhCO), 8,38 (s *él.,* 1 H, NH).
IR (NaCl): 3050 (ν_{C-H} arom.), 2924 (ν_{C-H} aliph), 1702 et 706 (ν_{C=O}) cm⁻¹.
UV (EtOH) : νₘₐₓ (v) 202 (51500), 220 (33300), 267 (15150) nm.
SM (*m*/*z* (%)) 464 (1, M^{.+}), 270 (58, M^{.+} - PhCO₂(CH₂)₄O), 177 (15, PhCO₂(CH₂)₄⁺), 105 (100, PhCO⁺).

| Analyse élémentaire pour C₂₇H₃₂N₂O₅ (464,57) : | |
|---|---|
| Calculée : | C, 69,81; H, 6,94; N, 6,03. |
| Trouvée : | C, 69,66; H, 7,02; N, 5,92. |

### C. Synthèse du 6-(3,5-diméthylbenzyl)-5-éthyl-1-(4-hydroxybutyloxyméthyl)-uracile.

Du 1-(4-benzoyloxybutyloxyméthyl)-6-(3,5-diméthylbenzyl)-5-éthyluracile (500 mg, 1,08 mmoles) a été additionné à une solution d'hydroxyde de sodium (360 mg, 9 mmoles) dans du méthanol (50 ml) et le mélange a été agité à 20°C pendant 3 h. Après neutralisation (acide chlorhydrique concentré) et concentration, le résidu a été soumis à une chromatographie sur colonne «flash» (dichlorométhane/méthanol 19:1) qui fournissait le 6-(3,5-diméthylbenzyl)-5-éthyl-1-(4-hydroxybutyloxyméthyl)uracile (190 mg, 48 %) sous la forme d'un solide blanc.
Point de fusion : 101,4-102,8 °C ;
R_{F} 0,29 (dichlorométhane/méthanol 19:1).
¹H RMN (CD₃OD, 200 MHz) : δ 1,00 (t, 3 H, J = 7,5 Hz, CH₂CH₃), 1,54 (m, 4 H, CH₂CH₂CH₂CH₂), 2,26 (s, 6 H, Me₂Ph), 2,41 (q, 2 H, CH₂CH₃), 3,52 (m, 4 H, CH₂CH₂CH₂CH₂), 4,11 (s, 2 H, CH₂Ph), 5,10 (s, 2 H, OCH₂N), 6,75 (s, 2 H, H*ₒᵣₜₕₒ*-benzyle), 6,90 (s, 1 H, H*ₚₐᵣₐ*-benzyle).
IR (KBr) 3395 (ν_{O-H}), 1694 et 1637 (ν_{C=O}) cm⁻¹.
UV (méthanol) : νₘₐₓ (v) 270 nm (11174).
SM (m/z (%)) : 360 (1, M^{.+}), 271 (7, M^{.+} - O(CH₂)₄OH), 258 (100, M^{.+} - HO(CH2)40CH2).

| Analyse élémentaire pour C₂₀H₂₈H₂O₄ (360,45) : | |
|---|---|
| Calculée : | C, 66,64; H, 7,83; N, 7,77. |
| Trouvée : | C, 66,39; H, 7,87; N, 7,63. |

### D. Synthès du 1-(4-N¹-hydroxyuréidobutyloxyméthyl)-6-(3,5-diméthylbenzyl)-5-éthyluracile (Composé comparatif Comp. 2)

A une solution de 6-(3,5-diméthylbenzyl)-5-éthyl-1-(4-hydroxybutyloxyméthyl)uracile (180 mg, 0,5 mmole), de *N*,*O*-*bis*(phénoxycarbonyl)hydroxylamine (150 mg, 0,55 mmole) et de triphénylphosphine (262 mg, 1 mmole) dans le tétrahydrofurane (5 ml), il a été ajouté, goutte à goutte à 0 °C, une solution de DIAD (diisopropylazodicarboxylate) (202 mg, 1 mmole) dans du tétrahydrofurane (81 ml). Le mélange réactionnel a alors été ramené à 20 °C, concentré et soumis à une chromatographie sur colonne de gel de silice (acétate d'éthyle/éther éthylique 1:9), puis à une chromatographie sur colonne «flash» (méthanol/chloroforme 3:97) pour conduire à 260 mg du composé intermédiaire 6-(3,5-diméthylbenzyl)-1-[4-(N-phénoxycarbonyl-*N*-phénoxycarbonyloxyamino)butyloxyméthyl]-5-éthyluracile

Ce composé intermédiaire a été immédiatement dissous dans une solution méthanolique saturée d'ammoniac (5 ml) et le milieu réactionnel agité à 20 °C pendant 24 h. A ce moment, le composé intermédiaire avait intégralement réagi (chromatographie sur couche mince) et le milieu réactionnel, concentré, a été soumis à une chromatographie sur colonne «flash» sur gel de silice (méthanol/acétate d'éthyle 1:9) pour fournir le 1-(4-*N*¹-hydroxyuréidobutyloxyméthyl)-6-(3,5-diméthylbenzyl)-5-éthyluracile (45 mg, 23 %) sous forme d'un solide.
Point de fusion : 148-150 °C,
R_{F} 0,40 (méthanol/acétate d'éthyle 1:9).
¹H RMN (CD₃OD, 200 MHz) : δ 1,00 (*t*, 3 H, J = 7,5 Hz, CH₂CH₃), 1,56 (*m*, 4 H, CH₂CH₂CH₂CH₂), 2,25 (*s*, 6 H, Me₂Ph), 2,41 (*q*, 2 H, CH₂CH₃), 3,42 et 3,51 (2 *t*, 2x2 H J = 6,25 Hz, NCH₂(CH₂)₂CH₂O et NCH₂(CH₂)₂CH₂O), 4,11 (s, 2 H, CH₂Ph), 5,10 (s, 2 H, OCH₂N), 6,72 (s, 2 H, H*ₒᵣₜₕₒ*-benzyle), 6,90 (s, 1 H, H*ₚₐᵣₐ*-benzyle).
IR (KBr) : 3485, 3356 et 3158 (ν_{NH} et ν_{OH}), 1682 et 1652 cm⁻¹ (ν_{C=O}).
SM (m/z (%)) : 373 (1,5, M^{.+}- NH₂COH), 355 (20, M^{.+} - NH₂COH - H₂O), 271 (40, M^{.+}- NH₂CONOH(CH₂)₄O), 258 (55, BH⁺), 257 (29, B⁺).

| Analyse élémentaire pour C₂₁H₃₀N₄O₅.H₂O (436,51) : | |
|---|---|
| Calculée : | C, 59,00; H, 7,30; N, 13,10. |
| Trouvée : | C, 59,40; H, 7,28; N, 13,09. |

### Exemple 9

### Synthèse du 1-(2-N¹-hydroxyuréidoéthoxyméthyl)-6-benzyl-5-éthyluracile (Composé comparatif Comp. 3)

### (Composé de Formule 1 comparatif dans lequel n = 2, R¹ = éthyle, R² = H, R³ = OH, R⁴ = H).

### A. Synthèse du 1-(2-acétoxyéthoxyméthyl)-6-benzyl-5-éthyluracile

A une suspension de 6-benzyl-5-éthyluracile (690 mg, 3 mmol), préparé comme décrit au point A de l'Exemple 1 en remplaçant l'acide 2-bromo-3-méthylbutanoïque par de l'acide 2-bromobutanoïque et le 3,5-diméthylphényléthanenitrile par du phényléthanenitrile, dans de l'HMDS (hexaméthyldisilazane) (15 ml, 75 mmoles), on a ajouté, sous couverture d'azote, du sulfate d'ammonium (15 mg). Le milieu réactionnel a été maintenu à ébullition sous reflux pendant 14 h, refroidi, puis concentré sous vide. Le résidu a été repris, sous couverture d'azote, par du dichlorométhane anhydre (12 ml). A la solution obtenue, on a ajouté dans des conditions strictement anhydres, du 1-acétoxy-2-acétoxyméthoxyéthane [A. Rosowski, S. H. Kim. M. Wick, J. Med. Chem. (1981) 24,1177-81] (1,06 g, 6 mmoles) et du triflate de triméthylsilyle (0,57 ml, 3,3 mmoles). Après 4 h d'agitation à 20 °C, on a ajouté du dichlorométhane (12 ml). La phase organique, lavée par une solution saturée d'hydrogénocarbonate de sodium (10 ml), puis par une solution saturée de chlorure de sodium (10 ml), séchée (sulfate de magnésium) et concentrée, a été soumise successivement à une chromatographie sur colonne «flash» de gel de silice (chloroforme/éthanol 9:1) puis à une seconde chromatographie sur colonne «flash» (éther de pétrole/acétate d'éthyle 1:1) pour fournir le 1-(2-acétoxyéthoxyméthyl)-6-benzyl-5-éthyluracile (380 mg, 69 %) qui a été immédiatement soumis à l'étape suivante.

### B. Synthèse du 6-benzyl-5-éthyl-1-(2-hydroxyéthoxyméthyl)uracile.

A une solution de 1-(2-acétoxyéthoxyméthyl)-6-benzyl-5-éthyluracile (1,74 g, 5,02 mmoles dans du méthanol (30 ml), une solution méthanolique 1 M de méthanolate de sodium (7 ml) a été ajoutée. Après 14 h d'agitation à 20 °C, la solution a été portée à pH 4 par une solution aqueuse 1 M d'acide chlorhydrique. Le milieu réactionnel concentré, soumis à une chromatographie sur colonne de gel de silice (chloroforme/éthanol 19:1) fournissait le 6-benzyl-5-éthyl-1-(2-hydroxyéthoxyméthyl)uracile (1 g, 66 %).
Point de fusion : 120-122 °C,
R_{F} 0,25 (chloroforme/éthanol 19:1),
¹H RMN (200 MHz, CDCl₃) : δ 0,88 (*t*, 3 H, J = 7,25 Hz, CH₂CH₃), 2,28 (*q*, 1 H, CH₂CH₃), 3,42 (*m*, 4 H, CH₂CH₂), 4,10 (*s él.,* 2 H, CH₂Ph), 4,65 (*t*, 1 H, J_{CH2,OH} = 4,5 Hz, CH₂OH), 5,01 (s, 2 H, OCH₂N), 7,25 (*s él*., 5 H, Ph), 11,45 (*s él,* 1 H, NH).
IR (KBr) 3396 (ν_{OH}), 3028 (ν_{C-H} arom.), 2832 (ν_{C-H} aliph.) 1706 cm⁻¹ (ν_{C=O}).

| Analyse élémentaire pour C₁₆H₂₀N₂O₄ (304,35) : | |
|---|---|
| Calculée : | C, 63,14; H, 6,62; N, 9,20. |
| Trouvée : | C, 62,94; H, 6,77; N, 9,07. |

### C. Synthèse du 1-(2-N¹-hydroxyuréidoéthoxyméthyl)-6-benzyl-5-éthyluracile (Composé comparatif Comp. 3)

A une solution de 6-benzyl-5-éthyl-1-(2-hydroxyéthoxyméthyl)uracile (150 mg, 0,4 mmole) dans du tétrahydrofurane (3 ml), on a ajouté à 20 °C de la triphénylphosphine (126 mg, 0,48 mmole et de la *N,O*-*bis*(phénoxycarbonyl)-hydroxylamine (120 mg, 0,44 mmole), puis, goutte à goutte, une solution de DIAD (diisopropylazodicarboxylate) (97 mg, 0,48 mmole) dans du tétrahydrofurane (0,5 ml). Le milieu réactionnel, concentré, a été soumis à une chromatographie sur colonne «flash» de gel de silice (chloroforme/éthanol 19:1) pour conduire à 300 mg du composé intermédiaire 6-benzyl-1-[2-(*N*-phénoxycarbonyl-*N-*phénoxycarbonyloxyamino)éthyloxyméthyl)-5-éthyluracile, qui a été immédiatement dissous dans une solution méthanolique saturée d'ammoniac (5 ml). Le milieu réactionnel a été agité 4 h à 20 °C, concentré, puis soumis à une chromatographie sur colonne «flash» de gel de silice (dichlorométhane/éthanol 9: 1) pour fournir, après recristallisation (acétate d'éthyle/méthanol) le 1-(2-*N*¹-hydroxyuréidoéthoxyméthyl)-6-benzyl-5-éthyluracile (80 mg, 45 %).
Point de fusion : 276-277 °C,
R_{F} 0,25 (dichlorométhane/méthane 9:1).
¹H RMN (200 MHz, (CD₃)₂SO, 80 °C) : δ 0,89 (*t*, 3 H, J = 7,3 Hz, CH₂CH₃), 2,30 (*q*, 2 H, CH₂CH₃), 3,47 (*t*, 2 H, J = 6,0 Hz, NCH₂CH₂), 3,59 (*t*, 2 H, CH₂CH₂O), 4,10 (*s él.*, 2 H, CH₂Ph), 5,03 (*s*, 2 H, OCH₂N), 6,00 (*s*, 2 H, NH₂), 7,22 (*s él.,* 5 H, Ph), 9,13 (*s*, 1 H, NOH), 11,16 (*s él.*, 1 H, NH).
IR (KBr) : 3475 et 3341 (ν_{NH} et v_{OH}), 1706 cm⁻¹ (ν_{C=O}).
SM (m/z (%)) 317 (3, M^{.+} - NH₂COH), 299 (6, M^{.+} - NH₂COH - H₂O), 243 (94, M^{.+} - NH₂CONOH(CH₂)₂O), 230 (100, M^{.+} - NH₂CONOH(CH₂)₂OCH).
UV [MeOH, νₘₐₓ (v)] : 205 (21210), 269 (11200) nm.

| Analyse élémentaire pour C₁₇H₂₂N₄O₅ (362,39) : | |
|---|---|
| Calculée : | C, 56,35; H, 6,12; N, 15,46. |
| Trouvée : | C, 55,89; H, 6,07; N, 15,20. |

## Revendications

1. Composé ayant la formule générale 1 suivante : 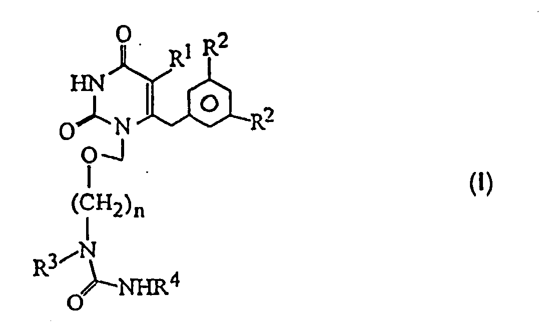 dans laquelle :
- n est égal à 3;
- R¹ représente un groupe éthyle ou un groupe isopropyle;
- chacun des groupes R² représente indépendamment l'un de l'autre un atome d'hydrogène, un groupe alkyle en C₁-C₃ ou un atome d'halogène;
- l'un des groupes R³ et R⁴ représente un atome d'hydrogène alors que l'autre des groupes R³ et R⁴ représente un groupe -OH ou -OR⁵, où R⁵ peut être un groupe acyle en C₂-C₇, un groupe alkyl(en C₁-C₆)aminocarbonyle, un groupe aralkyl(C₁-C₆)aminocarbonyle éventuellement substitué sur l'aryle, un groupe arylcarbonyle éventuellement substitué ou un groupe hétéroarylaminocarbonyle;
ou un sel pharmaceutiquement acceptable de celui-ci,

2. Composé selon la revendication 1, dans lequel R¹ représente un groupe isopropyle.

3. Composé selon la revendication 1 ou 2, dans lequel chaque R² représente un groupe méthyle.

4. Composé selon l'une quelconque des revendications 1 à 3, dans lequel R³ représente un groupe -OR⁵ et R⁴ représente un atome d'hydrogène.

5. Composé selon l'une quelconque des revendications 1 à 3, dans lequel R³ représente un groupe -OH et R⁴ représente un atome d'hydrogène.

6. Composé selon l'une quelconque des revendications 1 à 4, dans lequel R⁵ représente un groupe acyle.

7. Composé selon la revendication 4, dans lequel R⁵ représente un groupe pivaloyle.

8. Composé selon la revendication 1, qui est le 1-(3-*N*¹-hydroxyuréidopropyloxyméthyl)-6-(3,5-diméthylbenzyl)-5-isopropyluracile.

9. Composé selon la revendication 1, qui est le 1-(3-*N*¹-acétoxyuréidopropyloxyméthyl)-6-(3,5-diméthylbenzyl)-5-isopropyluracile.

10. Composé selon la revendication 1, qui est le 1-(3-*N*¹-pivaloyloxyuréidopropyloxyméthyl)-6-(3,5-diméthylbenzyl)-5-isopropyluracile.

11. Composé selon l'une quelconque des revendications 1 à 10, pour utilisation comme médicament.

12. Composé selon l'une quelconque des revendications 1 à 10, pour utilisation comme agent antiviral.

13. Composé selon la revendication 12, pour utilisation comme agent anti-HIV-1.

14. Procédé de préparation d'un composé de formule générale 1 suivante : 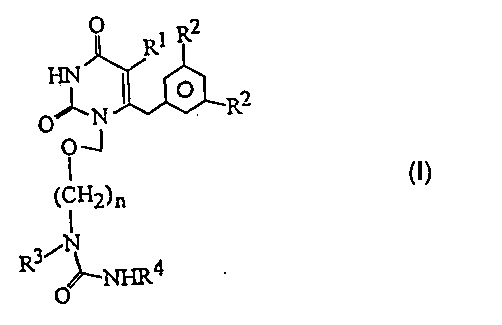 dans laquelle :
- n est égal à 3;
- R¹ représente un groupe éthyle ou isopropyle;
- chacun des groupes R² représente indépendamment l'un de l'autre un atome d'hydrogène, un groupe alkyle en C₁-C₃ ou un atome d'halogène;
- l'un des groupes R³ et R⁴ représente un atome d'hydrogène alors que l'autre des groupes R³ et R⁴ représente un groupe -OH ou -OR⁵, où R⁵ peut être un groupe acyle en C₂-C₇, un groupe alkyl(en C₁-C₆)aminocarbonyle, un groupe aralkyl(C₁-C₆ )aminocarbonyle éventuellement substitué sur l'aryle, un groupe arylcarbonyle éventuellement substitué ou un groupe hétéroarylaminocarbonyle; qui comprend les étapes consistant à :
a) faire réagir un composé de formule II suivante : 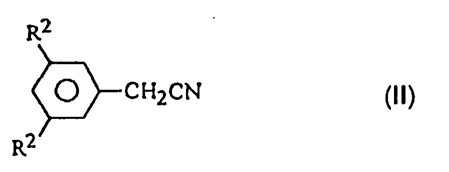 dans laquelle R² est comme défini ci-dessus, avec un composé de Formule III suivante :
R¹CHBrCOOEt (**III**)
dans laquelle R¹ est comme défini ci-dessus, en présence de Zn, pour obtenir un composé de Formule **IV** suivante : 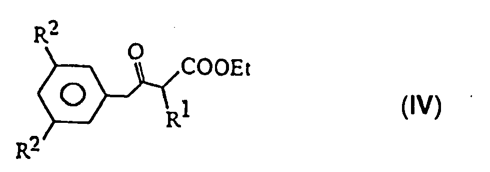 dans laquelle R¹ et R² sont comme définis ci-dessus;
b) faire réagir le composé de Formule **IV** obtenu dans l'étape a) ci-dessus avec de la thiourée dans un milieu alcoolique basique pour obtenir un thiouracile de Formule V suivante : 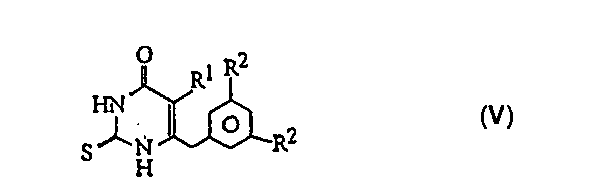 dans laquelle R¹ et R² sont tels que définis ci-dessus;
c) faire réagir le thiouracile de Formule V obtenu à l'étape b) ci-dessus avec un acide organique pour obtenir un composé de Formule VI suivante: 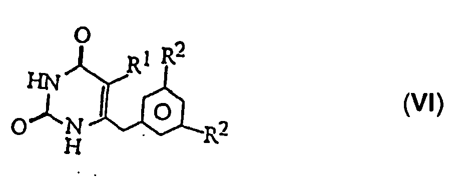 dans laquelle R¹ et R² sont tels que définis ci-dessus;
d) effectuer une réaction de glycosidation du composé de Formule VI obtenu à l'étape c) ci-dessus avec un composé de Formule VII suivante
MeCOOCH₂O(CH₂)ₙOCOMe (**VII**)
dans laquelle n est égal à 3, pour obtenir un ester, puis solvolyser l'ester pour fournir l'alcool de Formule IX suivante : 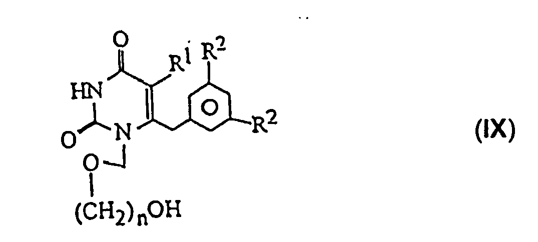 dans laquelle n, R¹ et R² sont tels que définis ci-dessus; et
e) soumettre l'alcool de Formule IX obtenu dans l'étape d) ci-dessus à une réaction de Mitsunobu en utilisant la N,O-*bis*(phénoxycarbonyl)hydroxylamine comme nucléophile pour fournir le composé de Formule X suivante : 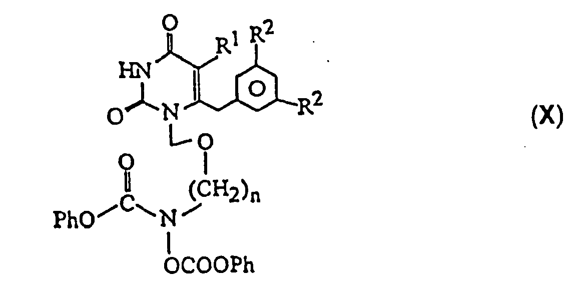 dans laquelle n, R¹ et R² sont tels que définis ci-dessus;
f) soumettre le composé de Formule X obtenu à l'étape e) ci-dessus à une aminolyse, pour fournir le composé de la présente invention de Formule la suivante : 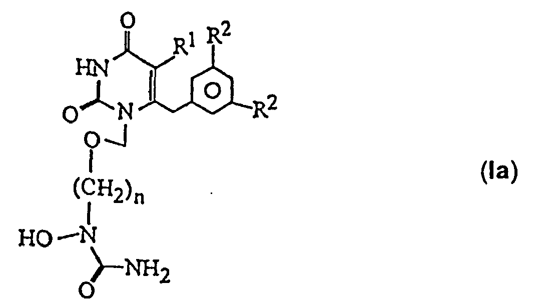 dans laquelle n, R¹ et R² sont comme définis ci-dessus; et
g) si nécessaire, protéger le groupe -OH du composé de Formule la obtenu dans l'étape f) ci-dessus avec un groupe R⁵ par une réaction d'O-acylation ou par une réaction d'O-carbamylation pour obtenir le composé de la présente invention de Formule **Ib** suivante : 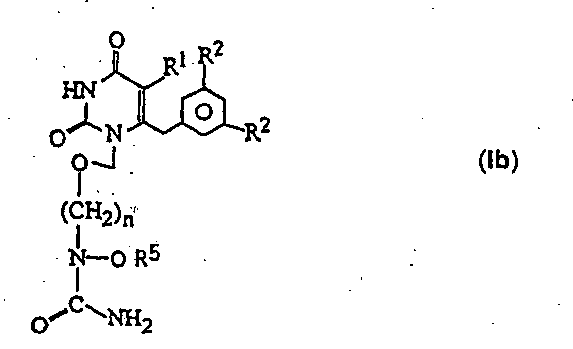 dans laquelle n, R¹, R² et R⁵ sont comme définis ci-dessus;
ou
h) soumettre l'alcool de Formule **IX** obtenu à l'étape c) ci-dessus à une réaction de Mitsunobu en utilisant le phthalimide comme nucléophile pour conduire à l'amine de Formule **XI** suivante : 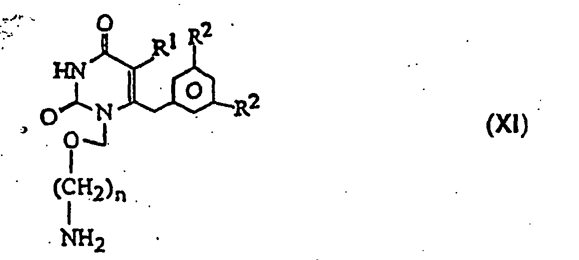 dans laquelle n, R¹ et R² sont comme définis ci-dessus,
i) traiter l'aminé de Formule **XI** obtenue à l'étape h) ci-dessus avec du carbonyldiimidazole et de la O-tertiobutyldiméthylsilylhydroxylamine pour founir le composé de Formule **XII** suivante : 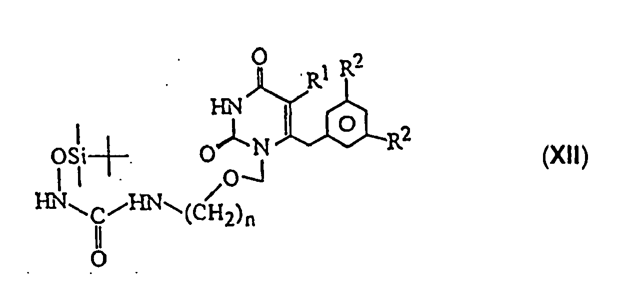 dans laquelle n, R¹ et R² sont définis comme ci-dessus;
j) désilyler le composé de Formule **XII** obtenu à l'étape i) ci-dessus pour obtenir le composé de Formule **Ic** suivante : 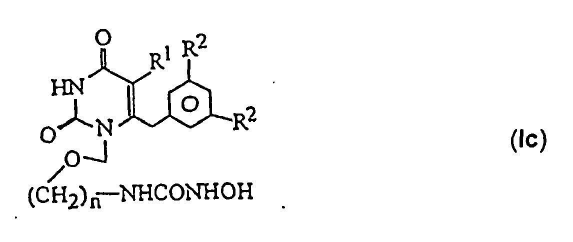 dans laquelle n, R¹ et R² sont comme définis ci-dessus;
k) si nécessaire, protéger le groupe -OH du composé de Formule **Ic** obtenu dans l'étape j) ci-dessus avec un groupe R⁵ par une réaction d'O-acylation ou par une réaction d'O-carbamylation pour obtenir le composé de Formule **Id** suivante : 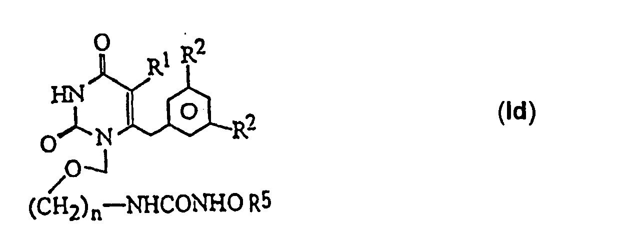
dans laquelle n, R¹, R² et R⁵ sont comme définis ci-dessus.

15. Composition pharmaceutique contenant en tant qu'ingrédient actif au moins un composé selon l'une quelconque des revendications 1 à 10 ou un sel pharmaceutiquement acceptable de celui-ci.

16. Composition pharmaceutique contenant une quantité efficace comme antiviral dudit composé selon l'une quelconque des revendications 1 à 10 ou un sel pharmaceutiquement acceptable de celui-ci.

17. Composition pharmaceutique selon la revendication 15 ou 16, pour utilisation comme médicament anti-VIH-1.

18. Utilisation d'un composé selon l'une quelconque des revendications 1 à 10, ou d'un sel pharmaceutiquement acceptable de celui-ci, pour la fabrication d'un médicament anti-VIH-1.

## Claims

1. A compound having the following general Formula I : 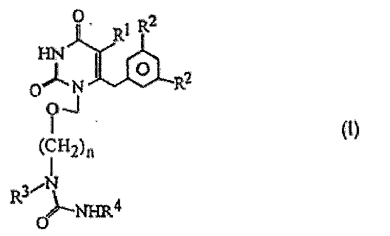 wherein :
- n is equal to 3;
- R¹ represents an ethyl group or an isopropyl group;
- each of the R² groups represents independently from each other a hydrogen atom, a C₁-C₃ alkyl group or a halogen atom;
- one of the R³ and R⁴ groups represents a hydrogen atom while the other of the R³ and R⁴ groups represents a -OH or -OR⁵ group, wherein R⁵ may be a C₂-C₇ acyl group, a C₁-C₆ alkylaminocarbonyl group, an ar(C₁-C₆)alkylaminocarbonyl group optionally substituted on the aryl, an arylcarbonyl group optionally substituted or a heteroarylaminocarbonyl group;
or a pharmaceutically acceptable salt thereof.

2. The compound according to Claim 1, wherein R¹ represents an isopropyl group.

3. The compound according to Claim 1 or 2, wherein each R² represents a methyl group.

4. The compound according to anyone of Claims 1 to 3, wherein R³ represents a -OR⁵ group and R⁴ represents a hydrogen atom.

5. The compound according to anyone of Claims 1 to 3, wherein R³ represents a -OH group and R⁴ represents a hydrogen atom.

6. The compound according to anyone of Claims 1 to 4, wherein R⁵ represents an acyl group.

7. The compound according to Claim 4, wherein R⁵ represents a pivaloyl group.

8. The compound according to Claim 1, which is the 1-(3-*N*¹-hydroxyureido-propyloxymethyl)-6-(3,5-dimethylbenzyl)-5-isopropyluracil.

9. The compound according to Claim 1, which is the 1-(3-*N*¹-acetoxyureido-propyloxymethyl)-6-(3,5-dimethylbenzyl)-5-isopropyluracil.

10. The compound according to Claim 1, which is the 1-(3-*N*¹-pivaloyloxyureido-propyloxymethyl)-6-(3,5-dimethylbenzyl)-5-isopropyluracil.

11. The compound according to anyone of Claims 1 to 10, for use as a medicament.

12. The compound according to anyone of Claims 1 to 10, for use as an antiviral agent.

13. The compound according to Claim 12, for use as an anti-HIV-1 agent.

14. A process for preparing a compound of the following general Formula 1 : 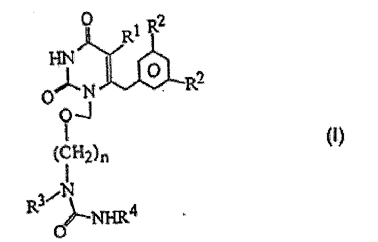 wherein :
- n is equal to 3;
- R¹ represents an ethyl or isopropyl group;
- each of the R² groups represents independently from each other a hydrogen atom, a C₁-C₃ alkyl group or a halogen atom;
- one of the R³ and R⁴ groups represents a hydrogen atom while the other of the R³ and R⁴ groups represents a -OH or -OR⁵ group, wherein R⁵ may be a C₂-C₇ acyl group, a C₁-C₆ alkylaminocarbonyl group, an ar(C₁-C₆)alkylaminocarbonyl group optionally substituted on the aryl, an arylcarbonyl group optionally substituted or a heteroarylaminocarbonyl group; which comprises the steps of :
a) reacting a compound of the following Formula **II** : 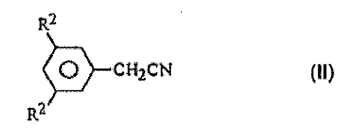 wherein R² is as defined above, with a compound of the following Formula III :
R¹CHBrCOOEt (**III**)
wherein R¹ is as defined above, in the presence of Zn, for obtaining a compound of the following Formula IV : 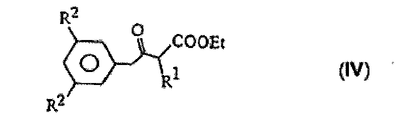 wherein R¹ and R² are as defined above;
b) reacting the compound of the Formula IV obtained in the above step a) with thiourea in a basic alcoholic medium for obtaining a thiouracil of the following Formula V : 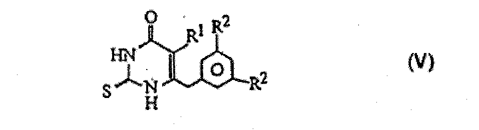 wherein R¹ and R² are as defined above;
c) reacting the thiouracil of the Formula V obtained in the above step b) with an organic acid for obtaining a compound of the following Formula **VI**: 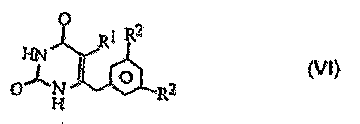 wherein R¹ and R² are as defined above;
d) carrying out a glycosidation reaction of the compound of the Formula **VI** obtained in the above step c) with a compound of the following Formula **VII**
MeCOOCH₂O(CH₂)ₙOCOMe (**VII**)
wherein n is equal to 3, for obtaining an ester, and then solvolysing the ester for providing the alcohol of the following Formula **IX** : 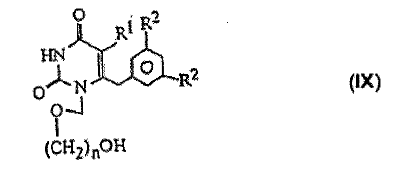 wherein n, R¹ and R² are as defined above; and
e) subjecting the alcohol of the Formula **IX** obtained in the above step d) to a Mitsunobu reaction by using *N*,*O*-*bis*(phenoxycarbonyl)hydroxylamine as a nucleophilic agent for providing the compound of the following Formula **X** : 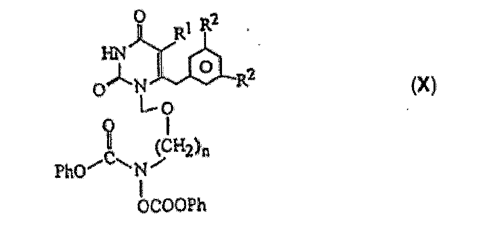 wherein n, R¹ and R² are as defined above;
f) subjecting the compound of the Formula X obtained in the above step e) to an aminolysis, for providing the compound of the present invention of the following Formula **Ia :** 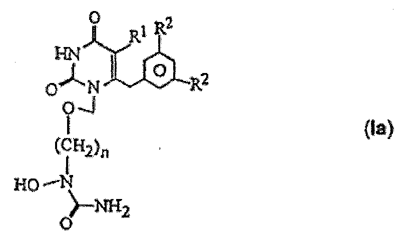 wherein n, R¹ and R² are as defined above; and
g) if required, protecting the -OH group of the compound of the Formula la obtained in the above step f) with a R⁵ group by a O-acylation reaction or by a O-carbamylation reaction for obtaining the compound of the present invention of the following Formula **Ib** : 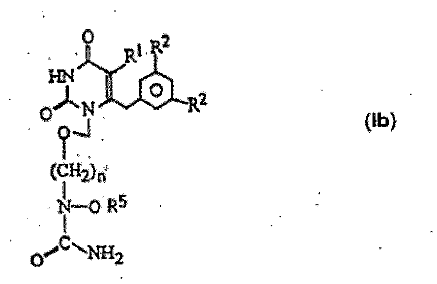 wherein n, R¹, R² and R⁵ are as defined above; or
h) subjecting the alcohol of the Formula **IX** obtained in the above step c) to a Mitsunobu reaction by using phthalimide as a nucleophilic agent for leading to the amine of the following Formula **XI** : 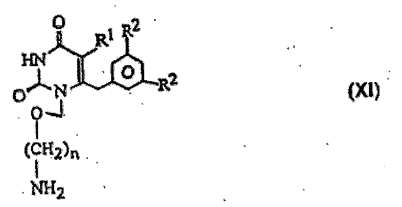 wherein n, R¹ and R² are as defined above,
i) treating the amine of the Formula **XI** obtained in the above step h) with carbonyldiimidazole and *O*-tertiobutyldimethylsilylhydroxylamine for providing the compound of the following Formula **XII :** 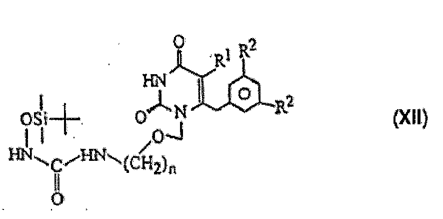 wherein n, R¹ and R² are as defined above;
j) desilylating the compound of the Formula **XII** obtained in the above step i) for obtaining the compound of the following Formula **Ic:** 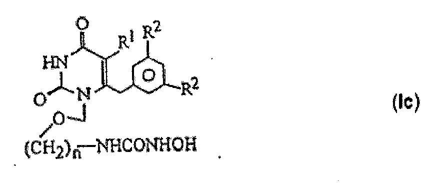 wherein n, R¹ and R² are as defined above;
k) if required, protecting the -OH group of the compound of the Formula **Ic** obtained in the above step j) with a R⁵ group by a O-acylation reaction or by a O-carbamylation reaction for obtaining the compound of the following Formula **Id** : 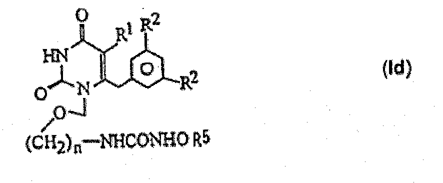
wherein n, R¹, R² and R⁵ are as defined above.

15. A pharmaceutical composition containing, as an active ingredient, at least one compound according to anyone of Claims 1 to 10 or a pharmaceutically acceptable salt thereof.

16. A pharmaceutical composition containing an antivirally effective amount of the compound according to anyone of Claims 1 to 10 or a pharmaceutically acceptable salt thereof.

17. The pharmaceutical composition according to Claim 15 or 16, for use as an anti-HIV-1 medicament.

18. Use of a compound according to anyone of Claims 1 to 10, or of a pharmaceutically salt thereof, for the manufacture of an anti-HIV-1 medicament.

## Patentansprüche

1. Verbindung mit der folgenden allgemeinen Formel 1: 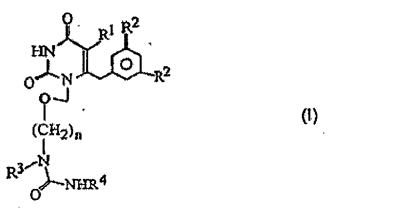 in der:
- n=3;
- R¹ eine Ethylgruppe oder Isopropylgruppe darstellt;
- jede der Gruppen R² unabhängig voneinander ein Wasserstoffatom, eine C₁- bis C₃-Alkylgruppe oder ein Halogenatom darstellt;
- eine der Gruppen R³ und R⁴ ein Wasserstoffatom darstellt, während die andere der Gruppen R³ und R⁴ eine -OH- oder -OR⁵-Gruppe darstellt, wobei R⁵ eine C₂- bis C₇-Acylgruppe, eine (C₁- bis C₆-Alkyl)aminocarbonylgruppe, eine eventuell am Aryl substituierte Ar(C₁- bis C₆-alkyl)aminocarbonylgruppe, eine eventuell substituierte Arylcarbonylgruppe oder eine Heteroarylaminocarbonylgruppe sein kann;
oder ein pharmazeutisch annehmbares Salz davon.

2. Verbindung nach Anspruch 1, in der R¹ eine Isopropylgruppe darstellt.

3. Verbindung nach Anspruch 1 oder 2, in der jedes R² eine Methylgruppe darstellt.

4. Verbindung nach einem der Ansprüche 1 bis 3, in der R³ eine -OR⁵-Gruppe und R⁴ ein Wasserstoffatom darstellen.

5. Verbindung nach einem der Ansprüche 1 bis 3, in der R³ eine -OH-Gruppe und R⁴ ein Wasserstoffatom darstellen.

6. Verbindung nach einem der Ansprüche 1 bis 4, in der R⁵ eine Acylgruppe darstellt.

7. Verbindung nach Anspruch 4, in der R⁵ eine Pivaloylgruppe darstellt.

8. Verbindung nach Anspruch 1, die das 1-(3-*N*¹-Hydroxyureidopropyloxymethyl)-6-(3,5-dimethylbenzyl)-5-isopropyluracil ist.

9. Verbindung nach Anspruch 1, die das 1-(3-*N*¹-Acetoxyureidopropyloxymethyl)-6-(3,5-dimethylbenzyl)-5-isopropyluracil ist.

10. Verbindung nach Anspruch 1, die das 1-(3-*N*¹-Pivaloyloxyureidopropyloxymethyl)-6-(3,5-dimethylbenzyl)-5-isopropyluracil ist.

11. Verbindung nach einem der Ansprüche 1 bis 10 zur Verwendung als ein Medikament.

12. Verbindung nach einem der Ansprüche 1 bis 10 zur Verwendung als ein Virostatikum.

13. Verbindung nach Anspruch 12 zur Verwendung als ein Mittel gegen HIV-1.

14. Verfahren zur Herstellung einer Verbindung der folgenden allgemeinen Formel **I**: 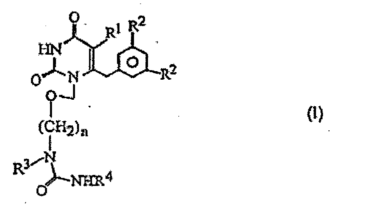 in der:
- n = 3;
- R¹ eine Ethyl- oder Isopropylgruppe darstellt;
- jede der Gruppen R² unabhängig voneinander ein Wasserstoffatom, eine C₁- bis C₃-Alkylgruppe oder ein Halogenatom darstellt;
- eine der Gruppen R³ und R⁴ ein Wasserstoffatom darstellt, während die andere der Gruppen R³ und R⁴ eine -OH- oder -OR⁵-Gruppe darstellt, wobei R⁵ eine C₂- bis C₇-Acylgruppe, eine (C₁- bis C₆-Alkyl)aminocarbonylgruppe, eine eventuell am Aryl substituierte Ar(C₁- bis C₆-alkyl)aminocarbonylgruppe, eine eventuell substituierte Arylcarbonylgruppe oder eine Heteroarylaminocarbonylgruppe sein kann;
das die Schritte umfasst, die daraus bestehen:
a) eine Verbindung der folgenden Formel **II**: 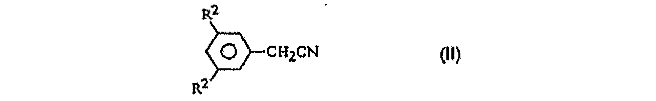 in der R² wie oben definiert ist, mit einer Verbindung der folgenden Formel **III**:
R¹CHBrCOOEt (**III**)
in der R¹ wie oben definiert ist, in Gegenwart von Zn umzusetzen, um eine Verbindung der folgenden Formel IV: 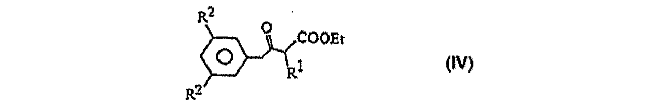 zu gewinnen, in der R¹ und R² wie oben definiert sind;
b) die im Schritt (a) oben gewonnene Verbindung der Formel IV in einem basischen alkoholischen Medium mit Thioharnstoff umzusetzen, um ein Thiouracil der folgenden Formel V: 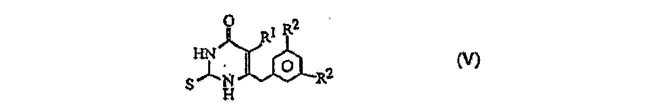 zu gewinnen, in der R¹ und R² wie oben definiert sind;
c) das im Schritt (b) oben gewonnene Thiouracil der Formel V mit einer organischen Säure umzusetzen, um eine Verbindung der folgenden Formel VI: 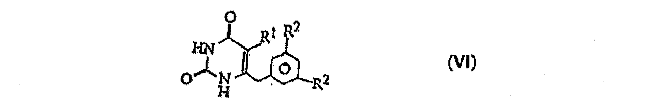 zu gewinnen, in der R¹ und R² wie oben definiert sind;
d) eine Glykosidierungsreaktion der im Schritt (c) oben gewonnenen Verbindung der Formel VI mit einer Verbindung der folgenden Formel VII:
MeCOOCH₂O(CH₂)ₙOCOMe (**VII**)
zu bewirken, in der n = 3, um einen Ester zu gewinnen, den Ester dann zu solvolysieren, um den Alkohol der folgenden Formel IX: 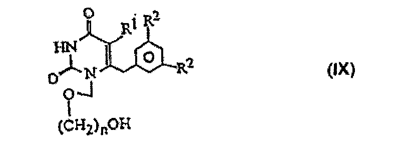 zu liefern, in dem n, R¹ und R² wie oben definiert sind; und
e) den im Schritt (d) oben gewonnenen Alkohol der Formel IX einer Mitsunobu-Reaktion zu unterwerfen, indem *N*,*O*-Bis(phenoxycarbonyl)hydroxylamin als Nucleophil verwendet wird, um die Verbindung der folgenden Formel X: 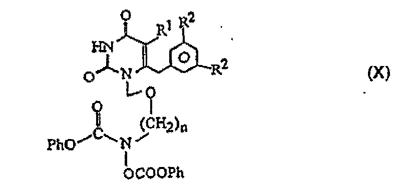 zu liefern, in der n, R¹ und R² wie oben definiert sind;
f) die im Schritt (e) oben gewonnene Verbindung der Formel X einer Aminolyse zu unterwerfen, um die Verbindung der vorliegenden Erfindung mit der folgenden Formel la: 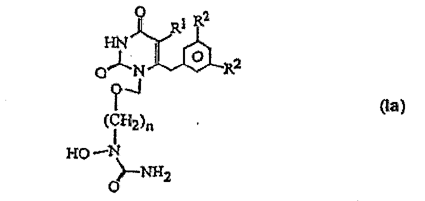 zu liefern, in der n, R¹ und R² wie oben definiert sind; und
g) wenn erforderlich, die-OH-Gruppe der im Schritt (f) oben gewonnenen Verbindung der Formel **Ia** durch eine O-Acylierungsreaktion oder durch eine O-Carbamylierungsreaktion mit einer Gruppe R⁵ zu schützen, um die Verbindung der vorliegenden Erfindung mit der folgenden Formel **Ib:** 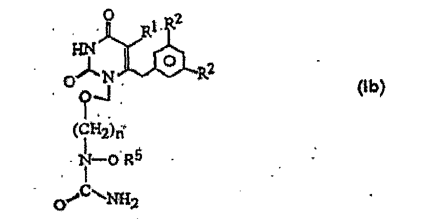 zu gewinnen, in der n, R¹, R² und R⁵ wie oben definiert sind; oder
h) den im Schritt (c) oben gewonnenen Alkohol der Formel IX unter Verwendung von Phthalimid als Nucleophil einer Mitsunobu-Reaktion zu unterwerfen, um zum Amin der folgenden Formel **XI**: 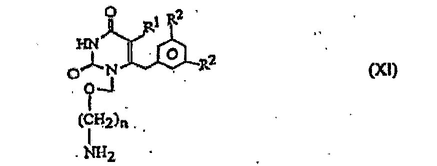 zu gelangen, in der n, R¹ und R² wie oben definiert sind,
i) das im Schritt (h) oben gewonnene Amin der Formel **XI** mit Carbonyldiimidazol und *O-tert*-Butyldimethylsilylhydroxylamin zu behandeln, um die Verbindung der folgenden Formel **XII:** 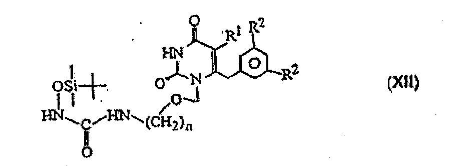 zu liefern, in der n, R¹ und R² wie oben definiert sind;
j) die im Schritt (i) oben gewonnnene Verbindung der Formel **XII** zu desilylieren, um die Verbindung der folgenden Formel **Ic**: 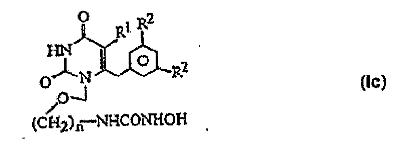 zu gewinnen, in der n, R¹ und R² wie oben definiert sind;
k) wenn erforderlich, die-OH-Gruppe der im Schritt (j) oben gewonnenen Verbindung der Formel **Ic** durch eine O-Acylierungsreaktion oder durch eine O-Carbamylierungsreaktion mit einer Gruppe R⁵ zu schützen, um die Verbindung der folgenden Formel **Id**: 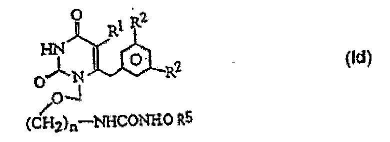
zu gewinnen, in der n, R¹, R² und R⁵ wie oben definiert sind.

15. Pharmazeutische Zusammensetzung, die als Wirkstoff zumindest eine Verbindung nach einem der Ansprüche 1 bis 10 oder ein pharmazeutisch annehmbares Salz davon enthält.

16. Pharmazeutische Zusammensetzung, die eine als Virostatikum wirksame Menge der Verbindung nach einem der Ansprüche 1 bis 10 oder eines pharmazeutisch annehmbaren Salzes davon enthält.

17. Pharmazeutische Zusammensetzung nach Anspruch 15 oder 16 zur Verwendung als ein Medikament gegen HIV-1.

18. Verwendung einer Verbindung nach einem der Ansprüche 1 bis 10 oder eines pharmazeutisch annehmbaren Salzes davon zur Herstellung eines Medikaments gegen HIV-1.
